(19) Europäisches Patentamt
European Patent Office
Office européen des brevets

(11) EP 0 736 056 B1

(12) **EUROPÄISCHE PATENTSCHRIFT**

(45) Veröffentlichungstag und Bekanntmachung des Hinweises auf die Patenterteilung:
**08.05.2002 Patentblatt 2002/19**

(21) Anmeldenummer: **95905095.6**

(22) Anmeldetag: **21.12.1994**

(51) Int Cl.7: **C08G 73/02**, A61K 49/04, A61P 9/00

(86) Internationale Anmeldenummer:
**PCT/EP94/04245**

(87) Internationale Veröffentlichungsnummer:
**WO 95/17448 (29.06.1995 Gazette 1995/27)**

(54) **KASKADENPOLYMERE MIT IODAROMATEN**

CASCADE POLYMERS WITH IODINE AROMATIC COMPOUNDS

POLYMERES EN CASCADE A COMPOSES AROMATIQUES D'IODE

(84) Benannte Vertragsstaaten:
**AT BE CH DE DK ES FR GB GR IE IT LI LU MC NL PT SE**

(30) Priorität: **22.12.1993 DE 4344464**

(43) Veröffentlichungstag der Anmeldung:
**09.10.1996 Patentblatt 1996/41**

(73) Patentinhaber: **SCHERING AKTIENGESELLSCHAFT**
**13353 Berlin (DE)**

(72) Erfinder:
- **KRAUSE, Werner**
  **D-13505 Berlin (DE)**
- **MAIER, Franz-Karl**
  **D-13467 Berlin (DE)**
- **BAUER, Michael**
  **D-13503 Berlin (DE)**
- **SCHUHMANN-GIAMPIERI, Gabriele**
  **D-12203 Berlin (DE)**
- **PRESS, Wolf-Rüdiger**
  **D-12103 Berlin (DE)**

(56) Entgegenhaltungen:
**EP-A- 0 430 863**
**WO-A-90/12050**
**WO-A-93/10824**
**WO-A-93/14147**
**WO-A-94/21600**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die in den Patentansprüchen gekennzeichneten Gegenstände, das heißt neue iodhaltige dendrimere Polymere, diese Verbindungen enthaltende Mittel, die Verwendung der polymeren Verbindungen als Kontrastmittel sowie Verfahren zur Herstellung dieser Verbindungen und Mittel.

Röntgenkontrastmittel sind unentbehrliche Hilfsmittel bei der Diagnose zahlreicher Erkrankungen wie z.B. von atherosklerotischen Gefäßprozessen, Tumoren, Infarkten, Erkrankungen der Nieren und ableitenden Harnwege und Perfusionsstörungen, z.B. am Herzen (Ischämie, sowie Entzündungen).

Die Anforderungen, die an derartige Kontrastmittel zu stellen sind, betreffen vor allem

a) eine ausreichend hohe Iodkonzentration
der verwendeten Lösung. Von ihr hängt - solange das Mittel nicht verdünnt wird - die Röntgendichte eines Kontrastmittels als einzigem Parameter ab. Das ist insbesondere in der Angiographie der Fall, wenn das Kontrastmittel mit hoher Geschwindigkeit über Katheter in Blutgefäße injiziert wird und damit das Blut verdrängt.

Bei einer Reihe von anderen Untersuchungen sind hochkonzentrierte Kontrastmittel ebenfalls erwünscht, z. B. wenn die Verdünnung im Körper sonst zu stark wird (Injektion in die Herzkammern, die Aorta oder bei der Intravenösen Digitalen Subtraktionsangiographie) oder bei ungünstigen Aufnahmebedingungen (so kann der Strahlengang durch den Körper eines schweren Patienten sehr lang werden);

b) die Chemotoxizität.
eine inhärente Eigenschaft der Kontrastmittel-Lösungen, die unter anderem verknüpft ist mit der Lipophilie der Moleküle, deren Protein-Affinität und Elektronendichte. Sie äußert sich in der klinischen Anwendung durch das Auftreten von Nebenwirkungen wie Übelkeit, Erbrechen, von bestimmten Kreislaufreaktionen, Urticaria, Bronchospasmus und anderen Symptomen bis hin zum Schock und Tod. Pharmakologisch sind chemotoxische Effekte z. B. als $LD_{50}$ nach intravenöser Injektion meßbar;

c) die Viskosität,
eine Größe, die für den Prozeß der Applikation der Kontrastmittel wichtig ist, z.B. wenn größere Volumina (30 - 100 ml) hochkonzentrierter und damit höher viskoser Lösungen rasch injiziert werden sollen. Außer der schlechten Injizierbarkeit haben höher viskose Kontrastmittel auch den Nachteil schlechter Mischbarkeit mit Blut (Schlierenbildung statt homogener Füllung der Herzhöhle oder Blutgefäße) und der Behinderung der Passage durch Kapillaren, z.B. der Lunge;

d) die Osmolalität
der Kontrastmittel-Lösungen. Im Falle der Applikation von gegenüber dem Blut und Gewebe stark hypertonen Lösungen (der physiologische Wert beträgt 310 m osm/kg) wird Wasser aus den Zellen getrieben, wodurch u.a. Zellmembranen zerstört und der Gesamtelektrolyt-Haushalt gestört wird. Hierdurch wird eine große Zahl von zum Teil schwerwiegenden Nebenwirkungen, wie z.B. Blutdruckabfall, Bradykardie bis hin zum Herzstillstand, Störungen der Blut-Hirn-Schranke, Gefäßschmerzen usw. verursacht;

e) eine Löslichkeit.
die für die praktische Anwendung der Kontrastmittel bei physiologischen pH-Werten in Wasser ausreichend hoch sein muß, ohne dabei jedoch gleichzeitig Verträglichkeit und Iodgehalt des Moleküls zu stark zu beeinträchtigen;

f) eine chemische Stabilität
der Kontrastmittel-Lösungen, die eine Hitzesterilisierung erlaubt, sowie eine Lagerfähigkeit von mindestens 24 Monaten ergibt.

Für die Darstellung von Gefäßen wären Röntgenkontrastmittel wünschenswert, die sich ausschließlich im vasalen Raum verteilen, d.h. das Verteilungsvolumen des Kontrastmittels sollte analog dem Intravasalraum sein. Die bisher für die Angiographie verwendeten Kontrastmittel sind mit dem Nachteil behaftet, daß sie den Vasalraum sehr schnell verlassen, weil sie zu klein und hydrophil sind und sich im Extrazellulärraum verteilen können. Darüberhinaus erfolgt ihre Ausscheidung so schnell, daß in der Regel eine lokale Applikation mittels Katheter (z.B. im cranialen Bereich) - behaftet mit vielen Unannehmlichkeiten für den Patienten - durchgeführt werden muß. Wünschenswert wären demnach blood pool agents (Perfusionsagentien), die es ermöglichen, nach systemischer Applikation mit Hilfe der Röntgenstrahltechnik gut durchblutetes von schlecht durchblutetem Gewebe abzugrenzen, um eine Ischämie zu diagnostizieren. Auch infarziertes Gewebe ließe sich aufgrund seiner Anämie vom umliegenden gesunden oder ischämischen Gewebe abgrenzen, wenn ein vasales Kontrastmittel angewandt wird. Dies ist von besonderer Bedeutung, wenn es z.B. darum geht, einen Herzinfarkt von einer Ischämie zu unterscheiden.

Eine weitere Anwendungsmöglichkeit besteht in der Diagnose von Gefäßbereichen mit verringerter oder erhöhter Permeabilität, die z.B. durch Entzündungen oder Tumore hervorgerufen sein kann, sowie in der Lymphographie und in der Mammographie.

Es besteht daher ein Bedarf an Röntgen-Kontrastmitteln, die den vasalen Raum markieren können (blood-pool-agent). Diese Verbindungen sollen sich durch eine gute Verträglichkeit sowie durch eine hohe Wirksamkeit (hohe Steigerung der Signalintensität bzw. Senkung der Dosis) und durch den Verbleib der Moleküle im vasalen Raum (keine Extravasation) sowie durch eine längere Halbwertszeit im Vergleich zu den für die Angiographie verwendeten Kontrastmitteln auszeichnen.

Der Ansatz, zumindest einen Teil dieser Probleme durch Verwendung von iodierten makromolekularen Kontrastmitteln zu lösen, war bisher nur sehr begrenzt erfolgreich.

So weisen die in der Internationalen Patentanmeldung WO 88/06162 beschriebenen Dextranderivate eine breite Molmassenverteilung und verbunden damit eine unvollständige Ausscheidung sowie eine unzureichende Verträglichkeit auf.

Die in der Internationalen Patentanmeldung WO 93/10824 offenbarten iodhaltigen Polyamine sind in Wasser nicht sehr gut löslich und zudem relativ schlecht verträglich.

Es bestand daher die Aufgabe, neue Röntgenkontrastmittel vor allem zur Erkennung und Lokalisierung von Gefäßkrankheiten, die die genannten Nachteile nicht besitzen, zur Verfügung zu stellen. Diese Aufgabe wird durch die vorliegende Erfindung gelöst.

Es wurde gefunden, daß sich iodhaltige dendrimere Polymere, die einen stickstoffhaltigen Kern und Triiodaromaten enthaltende und aliphatische Carboxy-, Sulfo- oder Phosphonogruppen tragende bildgebende Reste aufweisen, überraschenderweise hervorragend zur Herstellung von Röntgen-Diagnostika ohne die genannten Nachteile aufzuweisen, eignen. Die erfindungsgemäßen iodhaltigen dendrimeren Polymere lassen sich durch die allgemeine Formel I beschreiben

$$A\text{-}(X)_b \qquad (I),$$

worin

A für einen stickstoffhaltigen Kern der Basismultiplizität b steht, wobei
b für die Ziffern 1 bis 8 steht und
X für einen aus

$$\sum_{k=0}^{n-1} 2^k$$

Reproduktionseinheiten S und maximal $2^n$ bildgebenden Resten Z zusammengesetzten Rest steht, worin

n die Anzahl der Generationen bestimmt und für die Ziffern 1 bis 10 steht,
S für einen Rest der Formel II steht

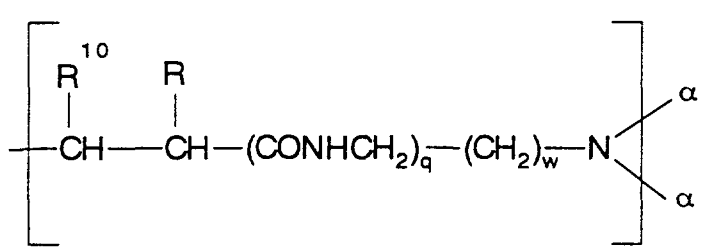

(II),

worin

R und $R^{10}$ unabhängig voneinander für ein Wasserstoffatom oder eine Methylgruppe,

w für die Ziffern 1 oder 2,

q für die Ziffern 0 oder 1 steht und die Positionen

α für $0 \leq k \leq n-1$ durch weitere Reproduktionseinheiten S besetzt werden und für die n-te Generation durch die Reste Z besetzt sind, wobei gegebenenfalls nicht durch Z besetzte Positionen α durch die Reste -(CO)q-U-COOH besetzt sind, worin q die oben angegebene Bedeutung hat und U für eine direkte Bindung oder eine Alkylenkette mit bis zu 6 C-Atomen, die gegebenenfalls durch 1 - 2 Sauerstoffatome unterbrochen und/oder gegebenenfalls durch 1 - 4 Hydroxygruppen und/oder durch 1 - 2 Carboxygruppen substituiert ist, stehen, mit der Maßgabe, daß maximal 20 % der Positionen α durch diesen Rest $-(CO)_q$-U-COOH besetzt sind,

Z aus einem Verknüpfungsglied Y und einem Triiodaromaten B bestehenden bildgebenden Rest Y-B be steht, wobei Y oder B eine aliphatische Carboxy-, eine Sulfo- oder Phosphonogruppe enthalten, und

Y für eine Gruppe -CO-, -CONH- -CSNH-,

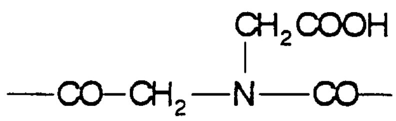 , 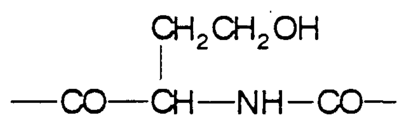 ,

-CHR-CHR-CONH-

oder

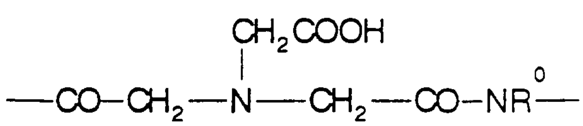

mit R in der oben genannten Bedeutung steht und $R^0$ in der Bedeutung eines Wasserstoffatoms, einer Methyl- oder einer Carboxymethylgruppe und

B für einen Benzolring

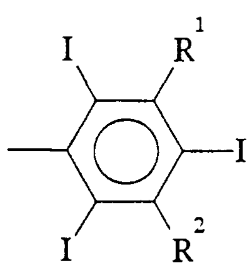

steht,
worin
$R^1$ und $R^2$ jeweils unabhängig voneinander ein Wasserstoffatom, eine $-CONR^3R^4$- oder $-NR^6COR^5$-Gruppe bedeuten,
wobei

$R^3$ und $R^4$ unabhängig voneinander für ein Wasserstoffatom, eine gegebenenfalls durch 1-5 Hydroxy- und/oder 1-3 $C_1$-$C_3$-Alkoxyund/oder 1 - 3 Carboxy-, Sulfo- oder

Phosphonogruppe(n) substituierte gerad- oder verzweigtkettige oder cyclische Alkylgruppe mit bis zu 12 C-Atomen,

$R^3$ und $R^4$ gemeinsam mit dem Stickstoffatom für einen gegebenenfalls ein Sauerstoffatom, eine $SO_2$-Gruppe oder einen N-CO-$R^7$-Rest - mit $R^7$ in der Bedeutung einer Carboxygruppe oder einer gegebenenfalls 1 - 5 Hydroxy-, 1-3 $C_1$-$C_3$-Alkoxy- oder 1 - 3 Carboxy-, Sulfo- oder Phosphonogruppe(n) enthaltenden Alkylgruppe mit bis zu12 C-Atomen - enthaltenden 5- oder 6-Ring,

$R^5$ für eine Carboxygruppe, eine gegebenenfalls durch ein Sauerstoffatom unterbrochene und/oder gegebenenfalls durch 1 - 3 Carboxy-, Sulfo- oder Phosphono- und/oder 1-5 Hydroxyund/oder 1 - 3 $C_1$-$C_3$-Alkoxygruppe(n) substituierte Alkylgruppe mit bis zu 12 C-Atomen,

$R^6$ für ein Wasserstoffatom, eine gegebenenfalls durch 1 - 3 Carboxy-, Sulfo- oder Phosphonogruppe(n) und/oder gegebenenfalls durch 1 - 3 Hydroxygruppe(n) und/oder 1 - 3 $C_1$-$C_3$-Alkoxygruppe(n) substituierte Alkylgruppe mit bis zu 12 C-Atomen

stehen, wobei die Reproduktionseinheiten S nur für eine Generation identisch sein müssen, sowie deren Salze mit physiologisch unbedenklichen organischen und/oder anorganischen Basen, Aminosäuren oder Aminosäureamiden.

Unter Dendrimeren versteht man verästelte polymere Moleküle, wie sie z.B. in Angew. Chem. Vol. 104, 1609 (1992) beschrieben sind.

Bevorzugt steht n für die Ziffern 2 bis 6.

Als Kaskadenkern A sind geeignet:

das Stickstoffatom, die Reste β-$NR^8$-β, β-$NR^8R^9$, die Reste der allgemeinen Formeln III, IV, V oder VI,

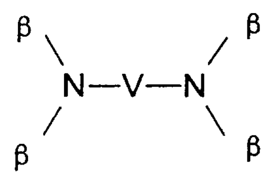

(III),

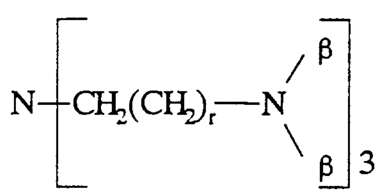

(IV),

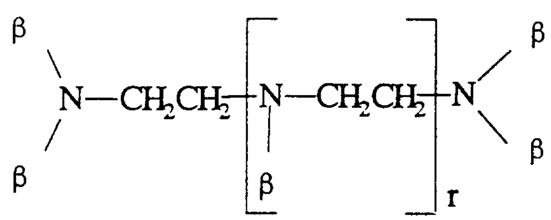

(V),

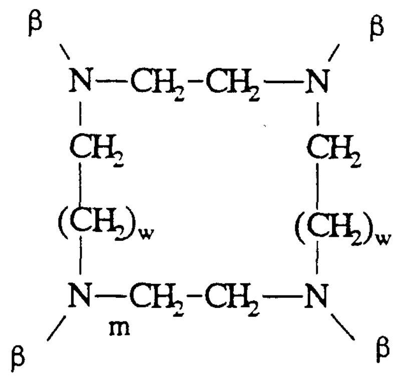

(VI),

worin

| | |
|---|---|
| $R^8$ und $R^9$ | unabhängig voneinander für einen geradkettigen oder verzweigten Alkyl-, Aryl- oder Aralkylrest mit bis zu 20 C-Atomen, der gegebenenfalls durch 1-4 Hydroxygruppe(n) substituiert ist, |
| β | die Bindungsstelle zum Rest X markiert, wobei die Anzahl der β mit der Basismultiplizität b gleichzusetzen ist, |
| V | für einen geradkettigen oder verzweigten Alkylen-, Arylen- oder Aralkylenrest mit bis zu 20 C-Atomen steht, der gegebenenfalls durch 1 - 4 Sauerstoffatom(e) unterbrochen und/oder durch 1-4 Hydroxygruppe (n) substituiert ist, |
| r | für die Ziffern 1, 2 oder 3, |
| w | für die Ziffern 1 oder 2 und |
| m | für die Ziffern 0, 1, 2 oder 3 steht. |

Die erfindungsgemäßen Polymere weisen ein Molekulargewicht von 5.000 bis 5.000.000. vorzugsweise 10.000 bis 500.000, besonders bevorzugt von 20.000 bis 100.000 auf.

Die Basismultiplizität b ist die Summe der freien Valenzen des stickstoffhaltigen Kerns und steht für die Ziffern 1 bis 8, vorzugsweise 1 bis 6.

Den einfachsten Fall eines Kaskadenkerns stellt das Stickstoffatom dar, dessen drei Bindungen (Basismultiplizität b = 3) in einer ersten "inneren Schicht" (Generation 1) von drei Reproduktionseinheiten S, die jeweils eine terminale $NH_2$-Gruppe trägt, besetzt sind (bzw. die drei Wasserstoffatome des zugrundeliegenden Kaskadenstarters Ammoniak sind durch drei Einheiten S substituiert worden). Die in einer nächsten Reaktionsfolge eingeführte 2. Schicht (Generation 2) von Reproduktionseinheiten S (die in dem oben genannten Beispiel mit A = Stickstoffatom 3 mal $2^1$ = sechs Bindungen besetzt) muß nicht identisch sein mit den Reproduktionseinheiten S der 1. Generation. Vorzugsweise sind die Reproduktionseinheiten S in allen Generationen eines Polymeren identisch. Nach maximal 10, vorzugsweise 2 bis 6, besonders bevorzugt 2 bis 4 Generationen, weist die äußerste Schicht b mal $2^n$ (im Falle des Stickstoffatoms als Kaskadenkern: 3 mal $2^n$) Positionen $\alpha$ an den terminalen Stickstoffatomen der letzten Generation auf, die zu 80 - 100 % durch die bildgebenden Reste Z und zu maximal 20 % durch die Reste $-(CO)_q$-U-COOH besetzt sind.

Als weitere bevorzugte Kaskadenstarter $A(H)_b$ seien beispielsweise aufgeführt:

| | |
|---|---|
| Tris-(2-aminoethyl)amin | (b = 6); |
| Tris-(3-aminopropyl)amin | (b = 6); |
| Diethylentriamin | (b = 5); |
| Triethylentetraamin | (b = 6); |
| Tetraethylenpentamin | (b = 7); |
| Tetramethylendiamin | (b = 4); |
| 1,4,7-Triazacyclononan | (b = 3); |
| 1,4,7,10-Tetraazacyclododecan | (b = 4); |
| 1,4,7,10,13-Pentaazacyclopentadecan | (b = 5); |
| 1,4,8,11-Tetraazacyclotetradecan | (b = 4); |
| 2-Hydroxy-1,3-propandiamin | (b = 4); |
| Xylylendiamin | (b = 4); |

(fortgesetzt)

| | |
|---|---|
| Hydroxyethylamin | (b = 2); |
| 2,3-Dihydroxypropylamin | (b = 2); |
| Methylamin | (b = 2); |
| Benzylamin | (b = 2); |
| Anilin | (b = 2); |
| Bis-(2,3-dihydroxylpropylamin) | (b = 1); |
| 2,3-Dihydroxypropylmethylamin | (b = 1); |
| Dibenzylamin | (b = 1); |
| 1,8-Diamino-3,6-dioxaoctan | (b = 4); |
| 1,5-Diamino-3-oxapentan | (b = 4). |

X steht für einen Zweig des dendrimeren Polymeren, der sich aus der Summe der Reproduktionseinheiten S und der dazugehörigen bildgebenden Resten Z ergibt.

So enthält z.B. ein aus n = 3 Generationen aufgebautes Polymeres insgesamt b mal

$$\sum_{k=0}^{3-1} 2^k$$

(= $2^\circ + 2^1 + 2^2$) = b mal 7 Reproduktionseinheiten S und weist b mal $2^3$ = b mal 8 terminale Positionen $\alpha$ auf, die von maximal b mal 8 bildgebenden Resten Z besetzt sind.

Bevorzugte Reproduktionseinheiten S sind

$-CH(CH_3)CH(CH_3)CONH-CH_2-CH_2-N$ ;

$-CH(CH_3)CH(CH_3)CH_2N$ ;

$-CH_2CH_2CH_2-N$ ;

$-CH_2CH(CH_3)CH_2-N$ ;

$-CH_2CH_2-CONH-CH_2CH_2-N$ ;

$-CH(CH_3)CH_2CH_2N$ ;

$-CH(CH_3)CH_2CONH-CH_2CH_2-N$ ;

$-CH_2CH(CH_3)CONH-CH_2-CH_2-N$.

Als im $R^1$- bzw. $R^2$-Substituenten des Triiodaromaten B enthaltene Alkylgruppen $R^3$, $R^4$ und $R^7$ kommen gerad- oder verzweigtkettige oder cyclische Kohlenwasserstoffe mit bis zu 12, vorzugsweise bis zu 10, besonders bevorzugt bis zu 6 C-Atomen in Betracht, die durch 1 - 5, vorzugsweise 1-3 Hydroxy- und/oder 1-3 $C_1$-$C_3$-Alkoxy- und/oder 1 - 3, vorzugsweise eine, Carboxy-, Sulfo- oder Phosphonogruppe(n) substituiert ist.

Namentlich genannt seien beispielsweise die Methyl-, Hydroxymethyl-, Ethyl-, 2-Hydroxyethyl-, 2-Hydroxy-1-(hydroxymethyl)-ethyl-, 1-(Hydroxymethyl)-ethyl-, Propyl-, Isopropyl-, 2-Hydroxypropyl-, 3-Hydroxypropyl-, 2,3-Dihydroxypropyl-, 1,2,3-Trihydroxypropyl-, Butyl-, Isobutyl-, 2-Hydroxybutyl-, 3-Hydroxybutyl-, 4-Hydroxybutyl-, 2-, 3- und 4-Hydroxy-2-methylbutyl-, 2- und 3-Hydroxyisobutyl-, 2,3,4-Trihydroxybutyl-, 1,2,4-Trihydroxybutyl-, Pentyl-, Cyclopentyl-, Cyclohexyl-, 2,3,4,5,6-Pentahydroxyhexyl-, 2-Methoxyethyl-, Carboxymethyl-, 2-Sulfoethyl-, Phosphonomethyl-, 2-Carboxyethyl-, 10-Hydroxydecyl-, Carboxy-, 3-Sulfopropyl-, 2-Phosphonoethyl-Gruppe.

Der durch $R^3$ und $R^4$ unter Einschluß des Amid-Stickstoffs gebildete heterocyclische 5- oder 6-Ring kann gegebenenfalls ein Sauerstoffatom, eine $SO_2$-Gruppe oder einen N-CO-$R^7$-Rest enthalten.

Als geeignete Heterocyclen seien beispielsweise genannt:
der Piperidyl-, Pyrazolidyl-, Morpholinyl-, N-substituierte Piperazinyl-, S,S-Dioxothiomorpholinyl-Ring.

Als im $R^1$- bzw. $R^2$-Substituenten des Triiodaromaten B enthaltene Reste $R^5$ und $R^6$ seien zusätzlich zu den entsprechenden für $R^3$, $R^4$, $R^7$ aufgeführten Reste die folgenden beispielhaft genannt:
Carboxymethoxymethyl-, 5-Carboxy-1,5-dihydroxy-3-oxapentyl-, 2-Carboxy-1-hydroxyethyl-, 3-Carboxy-2-oxapropyl-Gruppe.

Der für $R^8$ und $R^9$ bzw. V stehende Alkyl-, Aryl- oder Aralkylrest bzw. Alkylen-, Arylen- oder Aralkylenrest kann geradkettig oder verzweigt sein und bis zu 20, vorzugsweise bis zu 12 C-Atome enthalten. Der $R^8$- und $R^9$-Substituent kann durch 1 - 4, vorzugsweise 1 - 2 Hydroxygruppe(n) substituiert, die für V stehende Kette (gegebenenfalls zusätzlich) durch 1-4, vorzugsweise 1 - 2 Sauerstoffatome unterbrochen sein. Beispielhaft genannt seien folgende Gruppen:
Ethylen, Butylen, 1-Methylpropylen, Propylen, 3,6-Dioxaoctylen, Xylylen, 2-Hydroxypropylen, 3-Oxapentylen.

Die für U stehende Alkylenkette kann bis zu 6, vorzugsweise bis zu 2 C-Atome aufweisen und gegebenenfalls durch 1-2 Sauerstoffatome unterbrochen und/oder gegebenenfalls durch 1-4, vorzugsweise 1-2 Hydroxygruppen und/oder 1 - 2 Carboxygruppe(n) substituiert sein. Als beispielhaft für die Reste $-(CO)_q$-U-COOH seien aufgeführt:
$-CO(CH_2)_2COOH$; $-COCOOH$; $-CO(CHOH)_2COOH$; $-COCH_2OCH_2COOH$; $-COCH_2COOH$; $-COCH(OCH_3)COOH$, $CH_2CH_2COOH$, $CH(CH_3)CH_2COOH$, $CH_2CH(CH_3)COOH$, $CH(CH_3)CH(CH_3)COOH$, wobei die Reste $CO(CH_2)_2COOH$, $COCH_2OCH_2COOH$, $CH_2CH_2COOH$ bevorzugt sind.

Die aciden Wasserstoffatome der im Polymeren enthaltenen Säuregruppen können ganz oder teilweise durch Kationen von anorganischen und/oder organischen Basen, Aminosäuren oder Aminosäureamiden ersetzt sein.

Geeignete Kationen anorganischer Basen sind beispielsweise das Lithium-, das Kalium-, das Calcium-, das Magnesium- und insbesondere das Natriumion. Geeignete Kationen organischer Basen sind unter anderem solche von primären, sekundären oder tertiären Aminen, wie z.B. Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die erfindungsgemäßen Verbindungen weisen die eingangs geschilderten gewünschten Eigenschaften auf. Sie enthalten die für ihre Verwendung als Röntgenkontrastmittel benötigte Anzahl von Triiodaromaten. Sie verteilen sich nur im vasalen Raum und können daher diesen mit Hilfe der Röntgendiagnostik markieren.

Der Iodgehalt der erfindungsgemäßen Verbindungen liegt mit durchschnittlich 40 % gegenüber anderen Iodaromaten enthaltenden Makromolekülen wie den in der WO 88/06162 beschriebenen Dextranderivaten (ca. 2 bis 35 %) zum Teil um ein Mehrfaches höher. Die erfindungsgemäßen Verbindungen lassen sich überraschenderweise im Gegensatz zu den in der WO 88/06162 beschriebenen Dextranderivaten in jedem Verhältnis mit Wasser mischen, was zu einer höheren Kontrastmittelkonzentration in den Blutgefäßen kurz nach Injektion führt und sich damit günstig auf die Abgrenzung der Blutgefäße auswirkt. Der für Nebenwirkungen wie Schmerzen, Schädigungen der Blutgefäße und Herz-Kreislauf-Störungen verantwortliche Wert der Osmolalität ist deutlich verringert und nicht mehr hyperosmolar wie sonst bei Röntgenkontrastmitteln häufig beobachtet (Beispiel 1j: 220 [mosmol/kg] bei 37 °C, 130 mg Iod/ml). Die Osmolalität der erfindungsgemäßen Verbindungen ist auch deutlich geringer als die der in der WO 88/06162 beschriebenen Dextranverbindungen (440 mosmol/kg bei 90 mg Iod/ml).

Die für die akute Verträglichkeit verantwortliche Chemotoxizität der erfindungsgemäßen Verbindungen (Beispiel 6c: i.v.-$LD_{50}$ Maus >5 g Iod/kg) ist sowohl im Vergleich zu den makromolekularen Kontrastmitteln auf der Basis von Kohlenhydraten (WO 88/06162) als auch zu den in der WO 93/10824 beschriebenen Beispielen auf der Basis von Polyaminen deutlich verbessert.

Im Vergleich zu den Makromolekülen auf Dextranbasis ist auch die Viskosität der erfindungsgemäßen Verbindungen deutlich geringer, was eine Bolusinjektion und damit deutlich bessere Abgrenzung der Blutgefäße gegenüber dem umliegenden Gewebe erlaubt (Beispiel 6c: 4.02 mPas bei 37 °C und 100 mg Iod/ml; Beispiel 10 der WO 88/06162: 26 mPas bei 37 °C und 90 mg Iod/ml).

Mit den erfindungsgemäßen Verbindungen ist es gelungen, Makromoleküle mit definiertem Molekulargewicht herzustellen. Solche, in ihrer Molekülgröße exakt definierten makromolekularen Kontrastmittel mit Iodaromaten waren bislang nicht zugänglich.

Bei den Makromolekülen auf Dextranbasis, z.B. Dextran 40 000 (Rheomacrodex®), handelt es sich um ein Gemisch von Makromolekülen verschiedener Größe, deren mittleres Molekulargewicht z.B. bei 40 000 Dalton liegt. In dieser Mischung liegen jedoch auch Dextranmoleküle vor, die größer als 50 000 bzw. 60 000 Dalton sind. Dieser Anteil

an hochmolekularen Dextranverbindungen mag zwischen 5 und 10 % der Gesamtmenge liegen. Wie aus der Literatur bekannt (G. Arturson and G. Wallenius, The renal clearance of dextran of different molecular sizes in normal humans, Scandinav. J. Clin. & Lab. Investigation 1: 81 - 86, 1964), werden Dextranmoleküle dieser Größe nicht mehr glomerulär filtriert, und die renale Clearance dieser Moleküle ist daher nahezu null. Auch die in den Patentschriften EP 0206551, EP 0436316 und die in den Beispielen 1, 2 und 3 der WO 93/10824 beschriebenen Verbindungen können aufgrund ihrer hochmolekularen Anteile nach i.v.-Gabe nicht vollständig ausgeschieden werden. Von einem Diagnostikum erwartet man aber, daß es nach intravenöser Injektion in einem kurzen Zeitraum vollständig aus dem Körper eliminiert wird. Die übrigen in der WO 93/10824 beschriebenen Verbindungen verlassen dagegen den Intravasalraum zu rasch und sind somit nicht als Perfusionsagentien geeignet. Mit den erfindungsgemäßen Verbindungen ist es überraschenderweise erstmals gelungen, iodhaltige Polymere zur Verfügung zu stellen, die den vasalen Raum nur langsam verlassen, aber gleichzeitig die Kapillaren der Niere noch passieren und damit vollständig ausgeschieden werden. Aufgrund der Molekülstruktur zeigen die erfindungsgemäßen Verbindungen in den ersten 15 Minuten nach intravenöser Applikation eine Blutkonzentration, die etwa vierfach höher ist als bei den extrazellulären Röntgenkontrastmitteln wie z.B. Ultravist® (Abbildung, Seite 58). Dabei befinden sich die Kaskadenpolymere im Körper nur im Vasalraum, d.h. das Verteilungsvolumen beträgt ca. 0,05 l/kg. Nach intravenöser Applikation von 300 mg Iod/kg bei der Ratte zeigten die erfindungsgemäßen Verbindungen eine vollständige Ausscheidung (Retention <1 % der Dosis, 14 Tage nach intravenöser Applikation). Damit ist es erstmals möglich, makromolekulare Kontrastmittel mit Triiod-aromaten körpergerecht herzustellen.

Die erfindungsgemäßen Kaskadenpolymere dienen als Kontrastmittel zur Darstellung der Gefäße mittels der Röntgendiagnostik. Es ist damit möglich, ischämisches Gewebe von normalem Gewebe zu unterscheiden. Aber auch andere Schädigungen der Blut-Gewebe-Schranke sind mit diesen Verbindungen zu erkennen. Bei Entzündungen und Tumoren im Gehirn ist die Blut-Him-Schranke so geschädigt, daß das Kontrastmittel das kranke Gewebe infiltrieren kann und somit das kranke Gewebe mit der Röntgendiagnostik erkennbar wird. Aufgrund der Undurchlässigkeit der intakten Blut-Hirn-Schranke auch für kleine, aber hydrophile Moleküle konnte man Entzündungen und Tumore auch schon mit dem niedermolekularen Ultravist® erkennen. Wendet man in diesen Fällen aber die erfindungsgemäßen Kaskadenpolymere an, kann man die Dosis um das Vierfache reduzieren; denn die Makromoleküle verteilen sich in einem vierfach kleineren Raum, nämlich nur im Vasalraum, d.h. um gleiche Konzentrationen im Blut zu erreichen, genügt ein Viertel der Dosis.

Gleichzeitig kann man mit den erfindungsgemäßen Verbindungen Perfusionsmessungen z.B. am Myokard durchführen, was mit den niedermolekularen Verbindungen wie Ultravist® nur begrenzt möglich war, da diese Moleküle rasch in den interstitiellen Raum "auslaufen". Das "Auslaufen" in das Interstitium führte bei den niedermolekularen Verbindungen auch oft zu einer Unschärfe des Bildes, welches sich durch die erfindungsgemäßen Verbindungen vermeiden läßt. Gleichzeitig kann die Meßzeit gegenüber den niedermolekularen Verbindungen stark verlängert werden.

Ein weiterer Vorteil der vorliegenden Erfindung liegt darin, daß nunmehr Makromoleküle mit unterschiedlich lipophilen oder hydrophilen Triiodarylresten zugänglich geworden sind. Dadurch ist die Möglichkeit gegeben, Verträglichkeit und Pharmakokinetik dieser Kaskadenpolymere durch verschieden substituierte Triiodarylreste zu steuern.

Der Herstellung der erfindungsgemäßen iodhaltigen dendrimeren Polymere erfolgt dadurch, daß man dendrimere Polymere der allgemeinen Formel I'

$$A\text{-}(X')_b \qquad (I'),$$

worin

A und b die in den Ansprüchen 1-3 angegebene Bedeutung haben und

X' die für X in Anspruch 1 angegebene Bedeutung hat, wobei jedoch im Unterschied zu X für die n-te Generation die Positionen $\alpha$ nicht durch die Reste Z und gegebenenfalls $-(CO)_q$-U-COOH, sondern durch Wasserstoffatome besetzt sind, mit Verbindungen der allgemeine Formel II

$$Y'\text{-}B' \qquad (II),$$

worin

Y' für einen eine Carbonyl-, Thiocarbonyl-, aktivierte Carbonyl- oder eine CHR=CR-Gruppe - mit R in der Bedeutung eines Wasserstoffatoms oder eine Methylgruppe - enthaltenden in Y umzuwandelnden Rest steht und

B' die für B angegebene Bedeutung eines Triiodaromaten hat, wobei in B enthaltene Carboxy- und Hydroxygruppen jedoch in geschützter Form vorliegen,

umsetzt und anschließend die gegebenenfalls nicht durch die Reste Z besetzten Positionen α mit einem den Rest $-(CO)_q$-U-COOH einführenden Reagenz acyliert bzw. alkyliert.

Als Beispiel für eine aktivierte Carbonylgruppe in den Resten Y' der Edukte der allgemeinen Formel II seien Anhydrid, p-Nitrophenylester, Lacton und Säurechlorid genannt. Beispielhaft genannt für

Y' seien die Reste COCl; NCO; NCS;

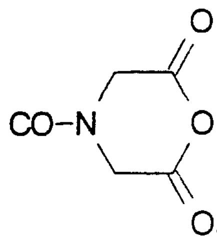

; ;

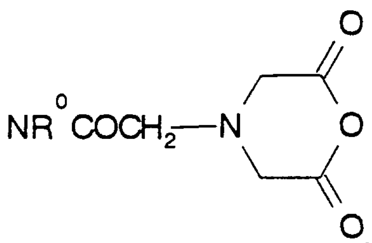

, ,

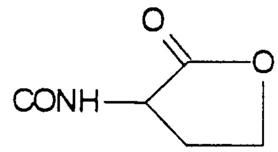

und NHCO-CR=CHR.

Als Säureschutzgruppen kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Abspaltung der Schutzgruppen erfolgt nach den dem Fachmann bekannten Verfahren [siehe z.B. E. Wünsch, Methoden der Org. Chemie (Houben Weyl), Bd. XV / 1, 4. Auflage 1974, S. 315 ff], beispielsweise durch Hydrolyse, Hydrogenolyse, alkalische Verseifung der Ester mit Alkali in wäßrig-alkoholischer Lösung bei Temperaturen von 0 bis 50 °C, saure Verseifung mit Mineralsäuren oder im Fall von z.B. tert.-Butylestern mit Hilfe von Trifluoressigsäure.

Als Hydroxyschutzgruppen kommen z.B. die Benzyl-, 4-Methoxybenzyl-, 4-Nitrobenzyl-, Trityl-, Diphenylmethyl-, Trimethylsilyl-, Dimethyl-t-butyl-silyl-, Diphenyl-tbutylsilylgruppe infrage.

Die Hydroxygruppen können auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen. Im Falle von Polyolen können die Hydroxygruppen auch in Form von Ketalen mit z.B. Aceton, Acetaldehyd, Cyclohexanon oder Benzaldehyd geschützt sein.

Bei gleichzeitigem Vorliegen von Carboxylgruppen können Hydroxygruppen auch durch intramolekulare Veresterung zu den entsprechenden Lactonen geschützt vorliegen.

Die Hydroxyschutzgruppen können nach den dem Fachmann bekannten Literaturmethoden, z.B. durch Hydrogenolyse, reduktive Spaltung mit Lithium/Ammoniak, Säurebehandlung der Ether und Ketale oder Alkalibehandlung der Ester freigesetzt werden (siehe z.B. "Protective Groups in Organic Synthesis", T.W. Greene, John Wiley and Sons 1981).

Die verschiedenen Verfahren zur Herstellung der erfindungsgemäßen Polymere sowie die dafür benötigten Ausgangsverbindungen sind dem Fachmann prinzipiell bekannt. Sie beruhen auf der Umsetzung der endständigen Aminogruppen der jeweils gewünschten Generation der dendrimeren Polymere der allgemeinen Formel I' mit den zur Generierung der an die Triiodaromaten B gebundenen Verknüpfungsglieder Y geeigneten Verbindungen der allgemeinen Formel II.

So erfolgt die Umsetzung von in Anhydridform vorliegenden N,N-bis(carboxymethyl)-amin- bzw. -amid-substituierten Triiodaromaten in flüssigen Reaktionsmedien wie beispielsweise Wasser, dipolaren aprotischen Lösungsmitteln wie Diethylether, Tetrahydrofuran, Dioxan, Acetonitril, N-Methylpyrrolidon, Dimethylformamid, Dimethylacetamid und dergleichen oder Gemische derselben unter Zusatz von Aminen, wie z.B. Triethylamin, N-Ethyldiisopropylamin, N,N-Dimethyl-4-aminopyridin. Die Reaktionstemperaturen liegen zwischen ca. -80 °C und 160 °C, wobei Temperaturen von 20 °C bis 80 °C bevorzugt sind. Die Reaktionszeiten liegen zwischen 0,5 Stunden und 7 Tagen, vorzugsweise zwischen 1 Stunde und 48 Stunden.

Die Herstellung der Säureanhydride kann nach bekannten Verfahren erfolgen, z.B. nach dem im US 3,660,388 bzw. in DE 16 95 050 beschriebenen Verfahren mit Acetanhydrid in Pyridin. In bestimmten Fällen ist es jedoch vorteilhaft, die Wasserabspaltung mit Carbodiimiden in einem geeigneten Lösungsmittel, wie z.B. Dimethylformamid oder Dimethylacetamid, schonend vorzunehmen.

Die Umsetzungen der isocyanat- bzw. isothiocyanat-substituierten Triiodaromaten erfolgt nach literaturbekannten Methoden (DOS 26 10 500, EP 0 431 838), beispielsweise in aprotischen Lösungsmitteln wie beispielsweise DMSO, DMF, DMA oder auch in Wasser bzw. wasserhaltigen Lösungsmittelgemischen bei Temperaturen von 0 - 120 °C, vorzugsweise 20 - 75 °C. Die Reaktionszeiten liegen in der Regel zwischen 1 - 48 Stunden, vorzugsweise 3 - 24 Stunden.

Die Umsetzung von Lactonstrukturen enthaltenden Triiodaromaten mit entsprechenden dendrimeren Polyaminen gelingt z.B. analog dem von T. Sheradsky, Y. Knobler und M. Frankel in J. Org. Chem., 26, 2710 (1961) beschriebenen Verfahren zur Aminolyse von 2-Acylamino-4-butyrolactonen.

Additionsreaktionen von einen olefinischen Substituenten CHR = CR-CONH aufweisenden Triiodaromaten werden z.B. nach der in Org. Synth. Coll. Vol. VI, S. 75 (1988) angegebenen Vorschrift durchgeführt, indem man ein triiodiertes Acrylamid in polaren Lösungsmitteln wie DMF, DMA, Pyridin, Ethanol mit dem gewünschten Polyamin umsetzt.

Die Acylierungen der terminalen Aminogruppen der Polymere der allgemeinen Formel I' mit Triiodaromaten, die einen Säurechlorid-Substituenten enthalten, werden nach den dem Fachmann bekannten Verfahren, z.B. analog der Vorschrift in EP 0015867 vorgenommen. Die Umsetzung wird im allgemeinen in polaren aprotischen Lösungsmitteln wie z.B. DMF, DMA, bzw. in Gemischen von polaren aprotischen Lösungsmitteln mit Wasser, in Gegenwart eines Säurefängers, wie z.B. tertiäres Amin (z.B. Triethylamin, Trimethylamin, N,N-Dimethylaminopyridin, 1,5-Diazabicyclo [4.3.0]nonen-5 (DBN), 1,5-Diazabicyclo[5.4.0]undecen-5 (DBU), Alkali-, Erdalkalicarbonat-, hydrogencarbonat oder -hydroxid (z.B. Kaliumcarbonat, Natriumcarbonat, Lithiumhydroxid, Kaliumhydroxid) bei Temperaturen zwischen 0 - 120 °C, vorzugsweise 20 - 80 °C, und Reaktionszeiten von 1 - 36 Stunden durchgeführt.

Die anschließende Acylierung bzw. Alkylierung der gegebenenfalls nicht an bildgebende Reste Y-B gebundenen terminalen Aminogruppen erfolgt analog literaturbekannten Vorschriften, siehe z.B. Org. Syn. Coll. Vol. 4, 5 (1963).

Die Neutralisation der Säuregruppen kann mit Hilfe anorganischer Basen (z.B. Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Omithin oder von Amiden ursprünglich neutraler oder saurer Aminosäuren durchgeführt werden.

Die als Edukte verwendeten dendrimeren Polymere der Formel I' werden analog literaturbekannten Methoden (z.B. Europäische Patentanmeldung EP 0 430 863; US-Patent 4 507 466; Internationale Anmeldung WO 93/14147) hergestellt.

Die bei den verschiedenen Verfahren eingesetzten iodierten Aromaten sind bekannt bzw. leicht aus bekannten generierbar.

So werden z.B. in den Deutschen Offenlegungsschriften DE 29 28 417 und DE 29 09 439 iodierte Aromaten beschrieben, die leicht mit z.B. Thionylchlorid zu den entsprechenden Säurechloridgruppen-haltigen Aromaten umgesetzt werden.

Isocyanat- bzw. isothiocyanat-substituierte Triiodaromaten können durch Umsetzung der entsprechenden Anilinderivate mit Phosgen bzw. Thiophosgen in aprotischen Lösungsmitteln wie z.B. 1,2-Dichlorethan, Dichlormethan, Pyridin, Dimethylsulfoxid, Tetrahydrofuran, Dioxan, Diethylether, Essigsäureethylester (Literatur: DOS 25 41 491) hergestellt werden.

Zu einem Lactonrest enthaltenden Triiodaromaten gelangt man zum Beispiel durch Umsetzung eines Triiodbenzoylchlorid-Derivats mit 2-Amino-4-butyrolacton-Hydrochlorid. Eine Umsetzung dieser Art wird z.B. von J. Brennan und P.J. Murphy in Tetrahedron Lett., 29 (17), 2063 (1988) beschrieben.

Triiodaromaten mit einem olefinischen Substituenten CHR=CR-CONH können analog den Angaben in WO 85/01727 erhalten werden.

Weitere aromatische Reste sind wie in M. Sovak; Radiocontrast Agents, Handbook of Experimental Pharmacology Vol. 73 (1984), Springer-Verlag, Berlin - Heidelberg - New York - Tokyo oder in der Europäischen Patentschrift EP 0 015 867, beschrieben, herstellbar.

Ein weiterer Gegenstand der Erfindung sind pharmazeutische Mittel, die mindestens eine der erfindungsgemäßen Verbindungen enthalten.

Die Erfindung betrifft weiterhin ein Verfahren zur Herstellung dieser Mittel, das dadurch gekennzeichnet ist,

daß die schattengebende Substanz mit den in der Galenik üblichen Zusätzen und Stabilisatoren in eine für die enterale bzw. parenterale Applikation geeignete Form gebracht wird. Die pharmazeutische Zubereitung kann im allgemeinen beliebig den spezifischen Bedürfnissen des Verwenders angepaßt werden. Die Konzentration der neuen Röntgenkontrastmittel im wäßrigen Medium richtet sich ganz nach der röntgendiagnostischen Methode. Der Iodgehalt der Lösungen liegt üblicherweise im Bereich zwischen 50 bis 450 mg/ml, vorzugsweise 70 bis 200 mg/ml.

Die resultierenden Mittel werden anschließend gewünschtenfalls hitzesterilisiert. Sie werden in Abhängigkeit des Iodgehalts und der verwendeten röntgendiagnostischen Methode bzw. Fragestellung in der Regel in einer Dosis von 30 mg Iod/kg bis 2000 mg I od/kg appliziert.

Die Applikation der wäßrigen Röntgenkontrastmittel-Lösung kann enteral oder parenteral, so oral, rektal, intravenös, intraarteriell, intravasal, intracutan, subcutan (Lymphographie), subarachnoidal (Myelographie) erfolgen.

Geeignete Zusätze sind beispielsweise physiologisch unbedenkliche Puffer (wie z.B. Tromethamin, Bicarbonat, Phosphat, Citrat), Stabilisatoren (wie z.B. DTPA, Natriumedetat, Calcium-dinatriumedetat), oder - falls erforderlich - Elektrolyte (wie z.B. Natriumchlorid) oder - falls erforderlich - Antioxidantien (wie z.B. Ascorbinsäure) oder auch Substanzen zur Anpassung der Osmolalität (wie z.B. Mannit, Glucose).

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoffen (z.B. Methylcellulose, Lactose, Mannit) und/oder Tensiden (z.B. Lecithine, Tweens®, Myrj® und/ oder Aromastoffen zur Geschmackskorrektur (z.B. ätherischen Ölen) gemischt.

Die nachfolgenden Beispiele dienen der näheren Erläuterung der Erfindungsgegenstände, ohne sie auf diese beschränken zu wollen.

**Beispiel 1**

Herstellung des 3-(3-Carboxypropionylamino)-5-(2,3-dihydoxypropylcarbamoyl)-2,4,6-triiodbenzoyl-Derivats des 48fachen primären Kaskadenamins [siehe Beispiel 1 h)]

a) Tris-[N,N-bis-(methoxycarbonylethyl)-2-aminoethyl]-amin

Zu 103,3 g (1,20 mol) Acrylsäuremethylester werden unter Rühren bei 20°C 14,69 g (0,100 mol) Tris-(2-aminoethyl)-amin, gelöst in 20 ml Methanol, zugetropft. Der Ansatz wird unter Argonatmosphäre 5 Tage bei Raumtemperatur und 2 Tage bei 50°C gerührt. Dann wird im Vakuum eingedampft und überschüssiger Acrylsäuremethylester durch azeotrope Destillation mit Toluol entfernt. Der Rückstand wird in 150 ml Methanol und 30 ml Diethylether aufgenommen, mit 150 ml Hexan ausgerührt und nach Abtrennen der Hexanphase im Vakuum eingedampft. Man erhält das Produkt als gelbliche Flüssigkeit, die ohne Reinigung weiter umgesetzt wird.
Ausbeute: 66,8 g (100 % d. Th.)

| Analyse (bezogen auf lösungsmittelfreie Substanz): | | | | |
|---|---|---|---|---|
| ber. | C 54,37 | H 8,21 | N 8,45 | O 28,97 |
| gef. | C 54,51 | H 8,13 | N 8,26 | |

b) Herstellung eines 6-fachen primären Kaskadenamines

Zu unter Argon bei Raumtemperatur gerührten 1363 ml (20,25 mol) Ethylendiamin wird innerhalb von 2 Stunden eine Lösung von 67,5 g (0,102 mol) des unter Beispiel 1a) hergestellten Hexamethylesters in 135 ml Methanol zugetropft. Nach 5-tägigem Rühren bei Raumtemperatur wird der Ansatz im Vakuum eingedampft. Der Rückstand wird in Methanol aufgenommen und 5 mal mit Diethylether gewaschen. Die methanolische Lösung wird im Vakuum eingedampft und der Rückstand am Hochvakuum getrocknet.
Ausbeute: 72,6 g (85,6 % d. Th.) gelbliches Öl

| Analyse (bezogen auf lösungsmittelfreie Substanz): | | | | |
|---|---|---|---|---|
| ber. | C 52,03 | H 9,46 | N 26,96 | O 11,55 |
| gef. | C 51,90 | H 9,62 | N 27,17 | |

c) Herstellung eines 12fachen dendrimeren Methylesters

Zu 559,5 g (6,28 mol) Acrylsäuremethylester wird unter Rühren bei 20°C eine Lösung von 43,73 g (0,053

mol) des unter Beispiel 1b) hergestellten Hexaamins in 120 ml Methanol zugetropft. Nach 5tägigem Rühren bei Raumtemperatur wird der Ansatz eingedampft und überschüssiger Acrylsäuremethylester wird durch azeotrope Destillation mit Toluol unter reduziertem Druck weitestgehend entfernt. Der Rückstand wird in wenig Methanol aufgenommen und mehrmals mit Diethylether/Hexan gewaschen. Das eingedampfte Rohprodukt wird durch Chromatographie an Kieselgel 60 (Merck) (Eluens: Methylenchlorid/Methanol) gereinigt. Nach Eindampfen der Produktfraktionen erhält man ein farbloses Öl.
Ausbeute: 95,6 g (96,8 % d. Th.)

| Analyse (bezogen auf lösungsmittelfreie Substanz): | | | | |
|---|---|---|---|---|
| ber. | C 54,12 | H 8,11 | N 12,02 | O 25,75 |
| gef. | C 53,93 | H 8,35 | N 11,84 | |

d) Herstellung eines 12fachen primären Kaskadenamins

Zu unter Argon bei Raumtemperatur gerührten 2,0 l(30 mol) Ethylendiamin wird innerhalb von 4 Stunden eine Lösung von 40,2 g (21,5 mmol) des unter Beispiel 1c) hergestellten Dodecamethylesters in 250 ml Methanol zugetropft. Der Ansatz wird 5 Tage bei Raumtemperatur gerührt, dann eingedampft, in wenig Methanol aufgenommen und mit Diethylether mehrmals gewaschen. Die methanolische Produktlösung wird im Vakuum eingedampft und der gelbliche, ölige Rückstand am Hochvakuum getrocknet.
Ausbeute: 47,3 g (100 % d. Th.)

| Analyse (bezogen auf lösungsmittelfreie Substanz): | | | | |
|---|---|---|---|---|
| ber. | C 52,39 | H 9,07 | N 25,46 | O 13,09 |
| gef. | C 52,18 | H 9,25 | N 25,63 | |

e) Herstellung eines 24fachen dendrimeren Methylesters

Zu 570 ml (6,30 mol) Acrylsäuremethylester wird unter Rühren bei Raumtemperatur eine Lösung von 40,9 g (18,6 mmol) des unter Beispiel 1d) hergestellten Dodecaamins in 180 ml Methanol zugetropft. Der Ansatz wird 6 Tage bei Raumtemperatur gerührt, dann im Vakuum eingedampft und überschüssiger Acrylsäuremethylester wird durch azeotrope Destillation mit Toluol unter reduziertem Druck weitestgehend entfernt. Der Rückstand wird in wenig Methanol aufgenommen und mehrmals mit Diethylether gewaschen. Die methanolische Lösung wird eingedampft und der Rückstand an Kieselgel 60 (Merck) (Eluens: Methanol/Pyridin) gereinigt. Nach Eindampfen der Produktfraktionen erhält man ein farbloses Öl.
Ausbeute: 73,4 g (92,5 % d. Th.)

| Analyse (bezogen auf lösungsmittelfreie Substanz): | | | | |
|---|---|---|---|---|
| ber. | C 54,05 | H 8,08 | N 13,13 | O 24,75 |
| gef. | C 54,27 | H 7,82 | N 13,02 | |

f) Herstellung eines 24fachen primären Kaskadenamins

Zu unter Argon bei Raumtemperatur gerührten 3,0 1 (45 mol) Ethylendiamin wird innerhalb von 4 Stunden eine Lösung von 72,7 g (17,0 mmol) der unter Beispiel 1e) hergestellten Kaskadenverbindung in 300 ml Methanol zugetropft. Der Ansatz wird 5 Tage bei Raumtemperatur gerührt, dann eingedampft, in wenig Methanol aufgenommen und mit Diethylether mehrmals gewaschen. Die methanolische Produktlösung wird im Vakuum eingedampft und der gelbliche, ölige Rückstand am Hochvakuum getrocknet.
Ausbeute: 82,6 g (98 % d. Th.)

| Analyse (bezogen auf lösungsmittelfreie Substanz): | | | | |
|---|---|---|---|---|
| ber. | C 52,51 | H 8,94 | N 24,95 | O 13,60 |
| gef. | C 52,44 | H 9,12 | N 25,21 | |

g) Herstellung eines 48fachen dendrimeren Methylesters

Zu 590,9 g (6,77 mol) Acrylsäuremethylester wird unter Rühren bei Raumtemperatur eine Lösung von 58,5 g (11,8 mmol) des unter Beispiel 1f) hergestellten Kaskadenamins in 200 ml Methanol zugetropft. Der Ansatz wird 7 Tage bei Raumtemperatur gerührt, dann im Vakuum eingedampft und überschüssiger Acrylsäuremethylester wird durch azeotrope Destillation mit Toluol unter reduziertem Druck weitestgehend entfernt. Der Rückstand wird in wenig Methanol aufgenommen und mehrmals mit Diethylether gewaschen. Die methanolische Lösung wird eingedampft und der Rückstand an Kieselgel 60 (Merck) (Eluens: Methanol/Pyridin) gereinigt. Nach Eindampfen der Produktfraktionen erhält man ein gelbliches Öl.
Ausbeute: 87,04 g (81,3 % d. Th.)

| Analyse (bezogen auf lösungsmittelfreie Substanz): | | | | |
|---|---|---|---|---|
| ber. | C 54,01 | H 8,07 | N 13,59 | O 24,34 |
| gef. | C 54,30 | H 7,86 | N 13,33 | |

h) Herstellung eines 48fachen primären Kaskadenamins

Zu unter Argon bei Raumtemperatur gerührten 3,0 l (45 mol) Ethylendiamin wird innerhalb von 4 Stunden eine Lösung von 78,4 g (8,64 mmol) der unter Beispiel 1g) hergestellten Kaskadenverbindung in 300 ml Methanol zugetropft. Der Ansatz wird 6 Tage bei Raumtemperatur gerührt, dann eingedampft, in wenig Methanol aufgenommen und mit Diethylether mehrmals gewaschen. Die methanolische Produktlösung wird im Vakuum eingedampft und der gelbliche ölige Rückstand am Hochvakuum getrocknet.
Ausbeute: 82,4 g (91,2 % d. Th.)

| Analyse (bezogen auf lösungsmittelfreie Substanz): | | | | |
|---|---|---|---|---|
| ber. | C 52,57 | H 8,88 | N 24,74 | O 13,82 |
| gef. | C 52,74 | H 8,93 | N 24,74 | |

i) 5-(3-Ethoxycarbonylpropionylamino)-2,4,6-triiodisophthalsäure-N-(2,3-diacetoxypropyl)-amid-chlorid

Zu einer unter Feuchtigkeitsausschluß gerührten Suspension von 73,4 g (100 mmol) 5-Amino-2,4,6-triiodisophthalsäure-N-(2,3-diacetoxypropyl)-amid-chlorid (EP 0308364) in 500 ml wasserfreiem Dioxan werden 24,7 g (150 mmol) Bernsteinsäurechlorid-monoethylester bei Raumtemperatur zugegeben. Der Ansatz wird mehrere Stunden am Rückfluß gekocht, bis laut Dünnschichtchromatographie kein Edukt mehr nachweisbar ist; dann wird eingedampft, der Rückstand in Dichlormethan aufgenommen und mit gesättigter wäßriger Natriumhydrogencarbonatlösung ausgeschüttelt. Die organische Phase wird nach Trocknen über wasserfreiem Magnesiumsulfat eingedampft und der Rückstand aus Essigsäureethylester/tert.-Butylmethylether umkristallisiert.
Ausbeute: 74,8 g (86,7 % d. Th.) farblose Kristalle

| Analyse (bezogen auf lösungsmittelfreie Substanz): | | | | | | |
|---|---|---|---|---|---|---|
| ber. | C 29,84 | H 2,57 | Cl 4,11 | I 44,14 | N 3,25 | O 16,69 |
| gef. | C 30,19 | H 2,63 | Cl 4,21 | I 44,07 | N 3,18 | |

j) Herstellung des 3-(3-Carboxypropionylamino)-5-(2,3-dihydoxypropylcarbamoyl)-2,4,6-triiodbenzoyl-Derivats des 48fachen primären Kaskadenamins [siehe Beispiel 1 h)]

Zu einer bei Raumtemperatur mechanisch gerührten Lösung von 39,6 g (45,9 mmol) des unter Beispiel 1i) hergestellten Säurechlorids in 200 ml N,N-Dimethylformamid wird eine Emulsion, bestehend aus 6,6 g (0,63 mmol) des unter Beispiel 1h) hergestellten Kaskadenamins, 9,54 ml (68,8 mmol) Triethylamin und 50 ml Wasser langsam zugetropft. Der Ansatz wird 2 Tage bei Raumtemperatur gerührt, dann mit 100 ml 2 n Natronlauge versetzt und 2 Stunden bei 50°C gerührt und anschließend wird nach Abkühlen auf Raumtemperatur mit 2 n Salzsäure neutralisiert und anschließend einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 24,7 g (87,3 % d. Th.) farbloses Lyophilisat

| Analyse (bezogen auf wasserfreie Substanz): | | | | | |
|---|---|---|---|---|---|
| ber. | C 31,61 | H 3,48 | I 40,89 | N 8,78 | O 15,25 |
| gef. | C 31,89 | H 3,62 | I 40,60 | N 8,95 | |

**Beispiel 2**

Herstellung des 3-Carboxymethylcarbamoyl-5-natriumcarboxylatomethylcarbamoyl-2,4,6-triiodphenylcarbamoyl-Derivats des 48fachen primären Kaskadenamins [siehe Beispiel 1 h)]

a) 5-Amino-2,4,6-triiodisophthalsäure-N,N'-bis-(methoxycarbonylmethyl)-diamid (EP 0129932)

Eine Lösung von 59,6 g (100 mmol) 5-Amino-2,4,6-triiodisophthalsäuredichlorid in 300 ml N,N-Dimethylformamid wird mit 27,6 g (220 mmol) Glycinmethylester-Hydrochlorid und 61,0 ml (440 mmol) Triethylamin versetzt. Es entsteht eine Suspension, die über Nacht bei Raumtemperatur unter Argon gerührt wird. Nach Eindampfen der Suspension am Vakuum wird der Rückstand aus Methanol umkristallisiert.
Ausbeute: 66,3 g (94,6 % d. Th.)

| Analyse (bezogen auf lösungsmittelfreie Substanz): | | | | | |
|---|---|---|---|---|---|
| ber. | C 23,99 | H 2,01 | I 54,31 | N 5,99 | O 13,69 |
| gef. | C 23,95 | H 2,14 | I 54,28 | N 6,09 | |

b) 5-Isocyanato-2,4,6-triiodisophthalsäure-N,N'-bis-(methoxycarbonylmethyl)-diamid

Zu einer bei 60°C Ölbadtemperatur unter Argonatmosphäre gerührten Suspension von 20,7 g (29,5 mmol) des unter Beispiel 2a) hergestellten Anilinderivates in 200 ml 1,2 -Dichlorethan werden 147 ml (73,8 mmol) einer zweinormalen toluolischen Phosgenlösung und 2 ml N,N-Dimethylformamid zugegeben. Nach vollständiger Umsetzung des Anilinderivates wird der Ansatz am Vakuum eingedampft, der Rückstand mit wasserfreiem Essigsäureethylester ausgerührt unter Stickstoffatmosphäre abgesaugt und am Ölpumpenvakuum getrocknet.
Ausbeute: 21,5 g (100 % d. Th.) hellbeiger Feststoff

| Analyse (bezogen auf lösungsmittelfreie Substanz): | | | | | |
|---|---|---|---|---|---|
| ber. | C 24,78 | H 1,66 | I 52,37 | N 5,78 | O 15,40 |
| gef. | C 24,82 | H 1,73 | I 52,35 | N 5,70 | |

c) Herstellung des 3-Carboxymethylcarbamoyl-5-natriumcarboxylatomethylcarbamoyl-2,4,6-triiodphenylcarbamoyl-Derivats des 48fachen primären Kaskadenamins [siehe Beispiel 1 h)]

Zu einer bei Raumtemperatur unter Argonatmosphäre gerührten Lösung von 18,7 g (22,7 mmol) des unter Beispiel 2b) hergestellten Isocyanats in 200 ml wasserfreiem Dimethylsulfoxid wird eine Lösung von 3,72 g (0,36 mmol) des unter Beispiel 1h) hergestellten Kaskadenamins in 37 ml wasserfreiem Dimethylsulfoxid langsam zugetropft. Der Ansatz wird 2 Tage bei Raumtemperatur gerührt, dann mit 30 ml 2 n Natronlauge versetzt und 2 Stunden bei 50°C gerührt. Die Lösung wird nach Abkühlen auf Raumtemperatur mit 2 n Salzsäure neutralisiert und dann einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 13,23 g (81,6 % d. Th.) gelbliches Lyophilisat

| Analyse (bezogen auf wasserfreie Substanz): | | | | | | |
|---|---|---|---|---|---|---|
| ber. | C 28,81 | H 2,81 | I 40,59 | N 10,20 | Na 2,45 | O 15,14 |
| gef. | C 29,10 | H 2,98 | I 40,28 | N 10,41 | Na 2,77 | |

**Beispiel 3**

Herstellung des 3,5-Bis-[(N-carboxymethyl)-natriumcarboxylatomethylcarbamoyl]-2,4,6-triiodphenylcarbamoyl-Derivats des 48fachen primären Kaskadenamins [siehe Beispiel 1 h)]

a) 5-Amino-2,4,6-triiodisophthalsäure-N,N,N',N'-tetrakis-(methoxycarbonylmethyl)-diamid

Eine Lösung von 47,2 g (79,2 mmol) 5-Amino-2,4,6-triiodisophthalsäuredichlorid in 250 ml N,N-Dimethylformamid wird mit 34,4 g (174 mmol) Iminodiessigsäuredimethylester-Hydrochlorid (Synthese nach Dubsky, Graenacher, Chem. Ber. 50, 1693(1917)) und 48,2 ml (348 mmol) Triethylamin versetzt. Es entsteht eine Suspension, die über Nacht bei Raumtemperatur unter Argon gerührt wird. Nach Eindampfen der Suspension am Vakuum wird der Rückstand in Dichlormethan aufgenommen und mit wäßriger Natriumhydrogencarbonatlösung ausgeschüttelt. Die organische Phase wird mit wasserfreiem Magnesiumsulfat getrocknet, filtriert und nach Einengen an Kieselgel 60 (Merck, Darmstadt) chromatographiert (Fließmittel: Dichlormethan/Methanol). Die Produktfraktionen werden im Vakuum zur Trockne eingedampft.
Ausbeute: 57,1 g (85, 3 % d. Th.) farbloser Feststoff

| Analyse (bezogen auf lösungsmittelfreie Substanz): | | | | | |
|---|---|---|---|---|---|
| ber. | C 28,42 | H 2,62 | I 45,05 | N 4,97 | O 18,93 |
| gef. | C 28,61 | H 2,77 | I 44,83 | N 4,72 | |

b) 5-Isocyanato-2,4,6-triiodisophthalsäure-N,N,N',N'-tetrakis-(methoxycarbonylmethyl)-diamid

Zu einer bei 60°C Ölbadtemperatur unter Argonatmosphäre gerührten Lösung von 30,6 g (36,2 mmol) des unter Beispiel 3a) hergestellten Anilinderivates in 300 ml 1,2-Dichlorethan werden 45,3 ml (90,5 mmol) einer zweinormalen toluolischen Phosgenlösung und 3 ml N,N-Dimethylformamid zugegeben. Nach vollständiger Umsetzung des Anilinderivates wird der Ansatz am Vakuum eingedampft, der Rückstand mit tert.-Butylmethylether ausgerühn, unter Stickstoffatmosphäre abgesaugt und am Ölpumpenvakuum getrocknet.
Ausbeute: 30,3 g (96,2 % d. Th.) rötlicher Feststoff

| Analyse (bezogen auf lösungsmittelfreie Substanz): | | | | | |
|---|---|---|---|---|---|
| ber. | C 28,95 | H 2,31 | I 43,70 | N 4,82 | O 20,20 |
| gef. | C 29,14 | H 2,46 | I 43,56 | N 4,73 | |

c) Herstellung des 3,5-Bis-[(N-carboxymethyl)-natriumcarboxylatomethylcarbamoyl]-2,4,6-triiodphenylcarbamoyl-Derivats des 48fachen primären Kaskadenamins [siehe Beispiel 1 h)]

Zu einer bei Raumtemperatur unter Argonatmosphäre gerührten Lösung von 28,8 g (33,1 mmol) des unter Beispiel 3b) hergestellten Isocyanats in 250 ml wasserfreiem Dimethylsulfoxid wird eine Lösung von 4,79 g (0,46 mmol) des unter Beispiel 1h) hergestellten Kaskadenamins in 48 ml wasserfreiem Dimethylsulfoxid langsam zugetropft. Der Ansatz wird 2 Tage bei Raumtemperatur gerührt, dann mit 66 ml 2 n Natronlauge versetzt und 2 Stunden bei 50°C gerührt. Die Lösung wird nach Abkühlen auf Raumtemperatur mit 2 n Salzsäure neutralisiert und dann einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 20,6 g (86,7 % d. Th.) gelbliches Lyophilisat

| Analyse (bezogen auf wasserfreie Substanz): | | | | | | |
|---|---|---|---|---|---|---|
| ber. | C 29,58 | H 2,73 | I 35,38 | N 8,90 | Na 4,27 | O 19,14 |
| gef. | C 29,84 | H 2,90 | I 35,11 | N 9,21 | Na 3,92 | |

**Beispiel 4**

Herstellung des 3-[(N-carboxymethyl)-methylcarbamoyl]-5-[(N-natriumcarboxylatomethyl)-methylcarbamoyl]-2,4,6-triiodphenylcarbamoyl-Derivats des 48fachen primären Kaskadenamins [siehe Beispiel 1 h)]

a) 5-Amino-2,4,6-triiodisophthalsäure-N,N'-bis-(ethoxycarbonylmethyl)-N,N'-dimethyldiamid

Eine Lösung von 59,6 g (100 mmol) 5-Amino-2,4,6-triiodisophthalsäuredichlorid in 300 ml N,N-Dimethylformamid wird mit 33,8 g (220 mmol) Sarcosinethylester-Hydrochlorid und 61,0 ml (440 mmol) Triethylamin versetzt. Es entsteht eine Suspension, die über Nacht bei Raumtemperatur unter Argon gerührt wird. Nach dem Eindampfen der Suspension am Vakuum wird der Rückstand in Dichlormethan aufgenommen und mit wäßriger Natriumhydrogencarbonatlösung ausgeschüttelt. Die organische Phase wird mit wasserfreiem Magnesiumsulfat getrocknet, filtriert und eingedampft. Der Rückstand wird aus Isopropanol umkristallisiert.
Ausbeute: 59,8 g (79 % d. Th.) farbloser Feststoff

| Analyse (bezogen auf lösungsmittelfreie Substanz): | | | | | |
|---|---|---|---|---|---|
| ber. | C 28,56 | H 2,93 | I 50,29 | N 5,55 | O 12,68 |
| gef. | C 28,73 | H 3,09 | I 50,02 | N 5,77 | |

b) 5-Isocyanato-2,4,6-triiodisophthalsäure-N,N'-bis-(ethoxycarbonylmethyl)-N,N'-dimethyldiamid

Zu einer bei 60°C Ölbadtemperatur unter Argonatmosphäre gerührten Lösung von 19,2 g (25,4 mmol) des unter Beispiel 4a) hergestellten Anilinderivates in 200 ml 1,2-Dichlorethan werden 31,7 ml (63,4 mmol) einer zweinormalen toluolischen Phosgenlösung und 2 ml N,N-Dimethylformamid zugegeben. Nach vollständiger Umsetzung des Anilinderivates wird der Ansatz am Vakuum eingedampft, der Rückstand mit tert.-Butylmethylether ausgerührt, unter Stickstoffatmosphäre abgesaugt und am Ölpumpenvakuum getrocknet.
Ausbeute: 19,26 g (97 % d. Th.) rötlicher Feststoff

| Analyse (bezogen auf lösungsmittelfreie Substanz): | | | | | |
|---|---|---|---|---|---|
| ber. | C 29,14 | H 2,57 | I 48,62 | N 5,37 | O 14,30 |
| gef. | C 29,08 | H 2,63 | I 48,50 | N 5,23 | |

c) Herstellung des 3-[(N-carboxymethyl)-methylcarbamoyl]-5-[(N-natriumcarboxylatomethyl)-methylcarbamoyl]-2,4,6-triiodphenylcarbamoyl-Derivats des 48fachen primären Kaskadenamins [siehe Beispiel 1 h)]

Zu einer bei Raumtemperatur unter Argonatmosphäre gerührten Lösung von 17,7 g (22,6 mmol) des unter Beispiel 4b) hergestellten Isocyanats in 150 ml wasserfreiem Dimethylsulfoxid wird eine Lösung von 3,27 g (0,31 mmol) des unter Beispiel 1h) hergestellten Kaskadenamins in 33 ml wasserfreiem Dimethylsulfoxid langsam zugetropft. Der Ansatz wird 2 Tage bei Raumtemperatur gerührt, dann mit 100 ml 2 n Natronlauge versetzt und 2 Stunden bei 50°C gerührt. Die Lösung wird nach Abkühlen auf Raumtemperatur mit 2 n Salzsäure neutralisiert und dann einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 13,1 g (91,3 % d. Th.) farbloses Lyophilisat

| Analyse (bezogen auf wasserfreie Substanz): | | | | | | |
|---|---|---|---|---|---|---|
| ber. | C 30,46 | H 3,14 | I 39,41 | N 9,91 | Na 2,38 | O 14,70 |
| gef. | C 30,61 | H 3,27 | I 39,18 | N 10,16 | Na 2,15 | |

**Beispiel 5**

Herstellung des 3-Carboxyformylamino-5-(2,3-dihydroxypropylcarbamoyl)-2,4,6-triiodbenzoyl-Derivats des 48fachen primären Kaskadenamins [siehe Beispiel 1 h)]

a) 5-(Ethoxycarbonylformylamino)-2,4,6-triiodisophthalsäure-N-(2,3-diacetoxypropyl)-amid-chlorid

Zu einer unter Feuchtigkeitsausschluß gerührten Suspension von 73,4 g (100 mmol) 5-Amino-2,4,6-triiodisophthalsäure-N-(2,3-diacetoxypropyl)-amid-chlorid (EP 0308364) in 300 ml wasserfreiem Dioxan werden 20,5 g (150 mmol) Oxalsäurechlorid-monoethylester bei Raumtemperatur zugegeben. Der Ansatz wird mehrere Stunden am Rückfluß gekocht bis laut Dünnschichtchromatographie kein Edukt mehr nachweisbar ist, dann wird eingedampft, der Rückstand in Dichlormethan aufgenommen und mit gesättigter wäßriger Natriumhydrogencarbonatlösung ausgeschüttelt. Die organische Phase wird nach Trocknen über wasserfreiem Magnesiumsulfat eingedampft und der Rückstand aus Essigsäureethylester/tert.-Butylmethylether umkristallisiert.
Ausbeute: 73,9 g (88,6 % d. Th.) farblose Kristalle

| Analyse (bezogen auf lösungsmittelfreie Substanz): | | | | | | |
|---|---|---|---|---|---|---|
| ber. | C 27,35 | H 2,17 | Cl 4,25 | I 45,62 | N 3,36 | O 17,25 |
| gef. | C 27,33 | H 2,28 | Cl 4,17 | I 45,49 | N 3,42 | |

b) Herstellung des 3-Carboxyformylamino-5-(2,3-dihydroxypropylcarbamoyl)-2,4,6-triiodbenzoyl-Derivats des 48fachen primären Kaskadenamins [siehe Beispiel 1 h)]

Zu einer bei Raumtemperatur mechanisch gerührten Lösung von 62,6 g (75,0 mmol) des unter Beispiel 5a) hergestellten Säurechlorids in 350 ml N,N-Dimethylformamid wird eine Emulsion bestehend aus 10,9 g (1,04 mmol) des unter Beispiel 1h) hergestellten Kaskadenamins, 15,6 ml (0,112 mol) Triethylamin und 80 ml Wasser langsam zugetropft. Der Ansatz wird 2 Tage bei Raumtemperatur gerührt, dann mit 150 ml 2 n Natronlauge versetzt und 2 Stunden bei 50°C gerührt. Die Lösung wird nach Abkühlen auf Raumtemperatur mit 2 n Salzsäure neutralisiert und dann einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 μm, Sartorius) filtriert und gefriergetrocknet.
Ausbeute: 41,9 g (93,0 % d.Th.) farbloses Lyophilisat

| Analyse (bezogen auf wasserfreie Substanz): | | | | | |
|---|---|---|---|---|---|
| ber. | C 29,93 | H 3,14 | I 42,16 | N 9,05 | O 15,73 |
| gef. | C 30,12 | H 3,42 | I 41,83 | N 9,27 | |

**Beispiel 6**

Herstellung des 3-(4-Carboxy-3-oxabutyrylamino)-5-(2,3-dihydroxypropylcarbamoyl)-2,4,6-triiodbenzoyl-Derivats des 48fachen primären Kaskadenamins [siehe Beispiel 1 h)]

a) Diglykolsäurechlorid-monoisopropylester

Zu 24,0 g (400 mmol) wasserfreiem Isopropanol werden unter Feuchtigkeitsausschluß 46,4 g (400 mmol) Diglykolsäureanhydrid gegeben. Die Temperatur der exothermen Reaktion wird mit einem Wasserbad auf 90 - 100°C moderiert. Nach 1 Stunde läßt man das Reaktionsgemisch abkühlen und versetzt es mit 32,0 ml (440 mmol) Thionylchlorid und 0,1 ml N,N-Dimethylformamid und läßt es 15 Stunden bei Raumtemperatur und 1 Stunde bei 50°C rühren. Die Titelverbindung wird durch Destillation bei 0,01 Torr und einer Siedetemperatur 100 - 101°C gewonnen.
Ausbeute: 67,6 g (86,8 % d.Th.) farblose Flüssigkeit
Gaschromatographie (100 %-Methode): Gehalt 96,4 %

| Analyse (bezogen auf lösungsmittelfreie Substanz): | | | | |
|---|---|---|---|---|
| ber. | C 43,20 | H 5,70 | Cl 18,22 | O 32,88 |
| gef. | C 43,34 | H 5,83 | Cl 18,01 | |

b) 5-[4-(2-Methylethyloxycarbonyl)-3-oxabutyrylamino]-2,4,6-triiodisophthalsäure-N-(2,3-diacetoxypropyl)-amid-chlorid

Zu einer unter Feuchtigkeitsausschluß gerührten Suspension von 73,4 g (100 mmol) 5-Amino-2,4,6-triiodisophthalsäure-N-(2,3-diacetoxypropyl)-amid-chlorid (EP 0308364) in 500 ml wasserfreiem Dioxan werden 29,2 g (150 mmol) das nach Beispiel 6a) hergestellten Säurechlorids bei Raumtemperatur zugegeben. Der Ansatz wird mehrere Stunden am Rückfluß gekocht, bis laut Dünnschichtchromatographie kein Edukt mehr nachweisbar ist, dann wird eingedampft, der Rückstand in Dichlormethan aufgenommen und mit gesättigter, wäßriger Natriumhydrogencarbonatlösung ausgeschüttelt. Die organische Phase wird nach Trocknen über wasserfreiem Magnesiumsulfat eingedampft und der Rückstand aus Essigsäureethylester/tert.-Butylmethylether umkristallisiert.
Ausbeute: 68,2 g (76,4 % d. Th.) farblose Kristalle

| Analyse (bezogen auf lösungsmittelfreie Substanz): | | | | | | |
|---|---|---|---|---|---|---|
| ber. | C 29,60 | H 2,71 | Cl 3,97 | I 42,65 | N 3,14 | O 32,88 |
| gef. | C 29,77 | H 2,83 | Cl 3,92 | I 42,41 | N 3,38 | |

c) Herstellung des 3-(4-Carboxy-3-oxabutyrylamino)-5-(2,3-dihydroxypropylcarbamoyl)-2,4,6-triiodbenzoyl-Derivats des 48fachen primären Kaskadenamins [siehe Beispiel 1 h)]

Zu einer bei Raumtemperatur mechanisch gerührten Lösung von 44,6 g (50,0 mmol) des unter Beispiel 6b) hergestellten Säurechlorids in 200 ml N,N-Dimethylformamid wird eine Emulsion bestehend aus 7,2 g (0,69 mmol) des unter Beispiel 1h) hergestellten Kaskadenamins, 10,4 ml (75,0 mmol) Triethylamin und 50 ml Wasser langsam zugetropft. Der Ansatz wird 2 Tage bei Raumtemperatur gerührt, dann mit 100 ml 2 n Natronlauge versetzt und 2 Stunden bei 50°C gerührt. Die Lösung wird nach Abkühlen auf Raumtemperatur mit 2 n Salzsäure neutralisiert und dann einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 31,49 g (83,7 % d.Th.) farbloses Lyophilisat

| Analyse (bezogen auf wasserfreie Substanz): | | | | | |
|---|---|---|---|---|---|
| ber. | C 31,07 | H 3,42 | I 40,20 | N 8,63 | O 16,68 |
| gef. | C 31,37 | H 3,65 | I 39,94 | N 8,82 | |

**Beispiel 7**

Herstellung des 3-[(N-Carboxymethyl)-natriumcarboxylatomethylcarbamoyl)-5-methoxyacetylamino-2,4,6-triiodbenzoyl-Derivats des 48fachen primären Kaskadenamins [siehe Beispiel 1 h)]

a) 5-Methoxyacetylamino-2,4,6-triiodisophthalsäure-N,N-bis-(methoxycarbonylmethyl)-amid-chlorid

Eine Lösung von 66,8 g (100 mmol) 5-Methoxyacetylamino-2,4,6-triiodisophthalsäuredichlorid (EP 0015867) in 300 ml wasserfreiem N,N-Dimethylformamid wird mit 21,75 g (110 mmol) Iminodiessigsäuredimethylester-Hydrochlorid (Synthese nach Dubsky, Graenacher, Chem. Ber. 50, 1693(1917)) und 30,5 ml (220 mmol) Triethylamin versetzt. Es entsteht eine Suspension, die 14 Stunden bei Raumtemperatur unter Argon gerührt wird. Der Ansatz wird in Dichlormethan aufgenommen, einmal mit Wasser, zweimal mit zweinormaler wäßriger Zitronensäure und einmal mit wäßriger Natriumhydrogencarbonatlösung ausgeschüttelt. Die organische Phase wird über wasserfreiem Magnesiumsulfat getrocknet und im Vakuum eingeengt. Durch Zutropfen von tert.-Butylether in die konzentrierte Lösung läßt sich die Titelverbindung als kristalliner Feststoff fällen, der abgesaugt und im Vakuum getrocknet wird.
Ausbeute: 57,4 g (72,4 % d.Th.)

| Analyse (bezogen auf lösungsmittelfreie Substanz): | | | | | | |
|---|---|---|---|---|---|---|
| ber. | C 25,76 | H 2,04 | Cl 4,47 | I 48,04 | N 3,54 | O 16,15 |
| gef. | C 25,82 | H 2,11 | Cl 4,48 | I 48,01 | N 3,38 | |

b) Herstellung des 3-[(N-carboxymethyl)-natriumcarboxylatomethylcarbamoyl)-5-methoxyacetylamino-2,4,6-triiodbenzoyl-Derivats des 48fachen primären Kaskadenamins (siehe Beispiel 1 h)]

Zu einer bei Raumtemperatur mechanisch gerührten Lösung von 36,3 g (45,8 mmol) des unter Beispiel 7a) hergestellten Säurechlorids in 200 ml N,N-Dimethylformamid wird eine Emulsion bestehend aus 6,6 g (0,64 mmol) des unter Beispiel 1h) hergestellten Kaskadenamins, 9,30 ml (68,7 mmol) Triethylamin und 50 ml Wasser langsam zugetropft. Der Ansatz wird 2 Tage bei Raumtemperatur gerührt, dann mit 100 ml 2 n Natronlauge versetzt und 2 Stunden bei 50°C gerührt. Die Lösung wird nach Abkühlen auf Raumtemperatur mit 2 n Salzsäure neutralisiert und dann einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 26,9 g (90,5 % d.Th.) farbloses Lyophilisat

| Analyse (bezogen auf wasserfreie Substanz): | | | | | | |
|---|---|---|---|---|---|---|
| ber. | C 30,43 | H 3,04 | I 39,37 | N 8,45 | Na 2.38 | O 16,34 |
| gef. | C 30,75 | H 3,27 | I 39,04 | N 8,58 | Na 2,06 | |

**Beispiel 8**

Herstellung des N-[3,5-Di-(acetylamino)-2,4,6-triiodbenzoyl]-N-(carboxymethyl)-glycyl-Derivats des 48fachen primären Kaskadenamins [siehe Beispiel 1 h)]

a) 3,5-Dinitrobenzoyl-N,N-bis-(carboxymethyl)-amid

133,1 g (1,00 mol) Iminodiessigsäure werden in 1,50 l zweinormaler Natronlauge gelöst und unter mechanischem Rühren mit 230,6 g (1,00 mol) 3,5-Dinitrobenzoylchlorid versetzt. Es entsteht eine dunkelrote Lösung, aus der die Titelverbindung durch Ansäuern mit halbkonzentrierter Salzsäure gefällt wird. Der Niederschlag wird abgesaugt, mit Wasser gewaschen und im Vakuum getrocknet.
Ausbeute: 260,5 g (79,6 % d. Th.) farblose Kristalle

| Analyse (bezogen auf wasserfreie Substanz): | | | | |
|---|---|---|---|---|
| ber. | C 40,38 | H 2,77 | N 12,84 | O 44,01 |
| gef. | C 40,42 | H 2,85 | N 12,63 | |

b) 3,5-Diaminobenzoyl-N,N-bis-(carboxymethyl)-amid

32,7 g (100 mmol) der unter Beispiel 8a) beschriebenen Dinitroverbindung werden in 500 ml Methanol vorgelegt, mit 1,6 g Palladium-Katalysator (10 % Palladium auf Aktivkohle) versetzt und unter Schütteln mit Wasserstoff hydriert. Nach Aufnahme der theoretischen Menge Wasserstoff wird vom Katalysator abfiltriert und zur Trockne eingedampft. Der Rückstand wird ohne Reinigung weiter umgesetzt.
Ausbeute: 26,7 g (100 % d. Th.) farbloser Feststoff

| Analyse (bezogen auf lösungsmittelfreie Substanz): | | | | |
|---|---|---|---|---|
| ber. | C 49,44 | H 4,90 | N 15,72 | O 29,93 |
| gef. | C 49,40 | H 4,98 | N 15,68 | |

c) 3,5-Diamino-2,4,6-triiodbenzoyl-N,N-bis-(carboxymethyl)-amid

24,7 g (92,4 mmol) der unter Beispiel 8b) hergestellten Verbindung werden in 200 ml Wasser und mit 150 ml einer zweinormalen Iodmonochlorid-Lösung innerhalb von 30 Minuten versetzt. Das Gemisch wird 12 Stunden bei Raumtemperatur gerührt und der gebildete Niederschlag abgesaugt. Der Feststoff wird in Wasser aufgeschlämmt, mit 10 g Natriumhydrogensulfit behandelt und erneut isoliert. Das Material wird in 300 ml Wasser durch Zugabe von 30 %-iger Natronlauge bei pH 8 gelöst, mit 2 g Aktivkohle versetzt, 5 Stunden gerührt und filtriert. Durch Ansäuern des Filtrats mit konzentrierter Salzsäure bildete sich ein Niederschlag, der abgesaugt und im Vakuum getrocknet wird.
Ausbeute: 40,1 g (67,3 % d. Th.) farbloser Feststoff

| Analyse (bezogen auf wasserfreie Substanz): | | | | | |
|---|---|---|---|---|---|
| ber. | C 20,49 | H 1,59 | I 59,03 | N 6,52 | O 12,40 |
| gef. | C 20,61 | H 1,63 | I 58,86 | N 6,68 | |

d) 3,5-Bis-(acetylamino)-2,4,6-triiodbenzoyl-N,N-bis-(carboxymethyl)-amid

38,6 g (59,9 mmol) der unter Beispiel 8c) hergestellten Verbindung werden in ein Gemisch aus 180 ml Essigsäureanhydrid und 0,5 ml konzentrierter Schwefelsäure eingetragen. Nach Rühren über Nacht bei Raumtemperatur wird Diethylether zugegeben und der gebildete Feststoff abfiltriert. Der Feststoff wird in 300 ml Wasser durch Zugabe von 30 %-iger Natronlauge bei pH 9 gelöst und anschließend durch Ansäuern mit konzentrierter Salzsäure bei pH 1 wieder ausgefällt. Der Niederschlag wird abgesaugt und im Vakuum getrocknet Ausbeute: 29,9 g (68,6 % d.Th.) farbloser Feststoff.

| Analyse (bezogen auf wasserfreie Substanz): | | | | | |
|---|---|---|---|---|---|
| ber. | C 24,71 | H 1,94 | I 52,22 | N 5,76 | O 15,36 |
| gef. | C 24,65 | H 2,03 | I 52,31 | N 5,65 | |

e) N-[3,5-Bis-(acetylamino)-2,4,6-triiodbenzoyl]-2,6-dioxomorpholin

28,1 g (38,5 mmol) der unter Beispiel 8d) dargestellten Verbindung werden in 56 ml wasserfreiem Pyridin gelöst, mit 7,3 ml (77 mmol) Essigsäureanhydrid versetzt und unter Feuchtigkeitsausschluß 10 Stunden bei Raumtemperatur gerührt. Durch Zutropfen von wasserfreiem Diethylether wird das gebildete Anhydrid gefällt, abfiltriert und im Vakuum getrocknet.
Ausbeute: 27,4 g (100 % d.Th.) hellbeiger Feststoff

| Analyse (bezogen auf lösungsmittelfreie Substanz): | | | | | |
|---|---|---|---|---|---|
| ber. | C 25,34 | H 1,70 | I 53,55 | N 5,91 | O 13,50 |
| gef. | C 25,21 | H 1,83 | I 53,58 | N 5,86 | O 13,73 |

f) Herstellung des N-[3,5-Di-(acetylamino)-2,4,6-triiodbenzoyl]-N-(carboxymethyl)-glycyl-Derivats des 48fachen primären Kaskadenamins [siehe Beispiel 1 h)]

Zu einer bei Raumtemperatur mechanisch gerührten Lösung von 25,6 g (36,0 mmol) des unter Beispiel 8e) hergestellten Anhydrids in 200 ml N,N-Dimethylformamid wird eine Emulsion bestehend aus 5,2 g (0,50 mmol) des unter Beispiel 1h) hergestellten Kaskadenamins, 7,5 ml (54,0 mmol) Triethylamin und 30 ml Wasser langsam zugetropft. Der Ansatz wird 2 Tage bei Raumtemperatur gerührt, dann mit 2 n Salzsäure neutralisiert und einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 19,5 g (87,5 % d. Th.)

| Analyse (bezogen auf wasserfreie Substanz): | | | | | |
|---|---|---|---|---|---|
| ber. | C 31,71 | H 3,38 | I 41,02 | N 10,31 | O 13,58 |
| gef. | C 31,98 | H 3,52 | I 40,76 | N 10,54 | |

**Beispiel 9**

Herstellung des 3-[(N-Carboxymethyl)-methoxyacetylamino-5-(2,3-dihydroxypropylcarbamoyl)-2,4,6-triiodbenzoyl-Derivats des 48fachen primären Kaskadenamins [siehe Beispiel 1 h)]

a) 5-Methoxyacetylamino-2,4,6-triiodisophthalsäure-N-(2,3-diacetoxypropyl)-amid-chlorid

Zu einer unter Feuchtigkeitsanschluß gerührten Suspension von 73,4 g (100 mmol) 5-Amino-2,4,6-triiodisophthalsäure-N-(2,3-diacetoxypropyl)-amid-chlorid (EP 0308364) in 500 ml wasserfreiem Dioxan werden 24,7 g (150 mmol) Methoxyacetylchlorid bei Raumtemperatur zugegeben. Der Ansatz wird mehrere Stunden am Rückfluß gekocht, bis lt. Dünnschichtchromatographie kein Edukt mehr nachweisbar ist, dann wird eingedampft, der Rückstand in Dichlormethan aufgenommen und mit gesättigter wäßriger Natriumhydrogencarbonatlösung ausgeschüttelt. Die organische Phase wird nach Trocknen über wasserfreiem Magnesiumsulfat eingedampft und der Rückstand aus Essigsäureethylester/tert.-Butylmethylether umkristallisiert.
Ausbeute: 73,2 g (90,7 % d.Th.) farblose Kristalle

| Analyse (bezogen auf lösungsmittelfreie Substanz): | | | | | | |
|---|---|---|---|---|---|---|
| ber. | C 26,81 | H 2,25 | Cl 4,40 | I 47,21 | N 3,47 | O 15,87 |
| gef. | C 26,79 | H 2,32 | Cl 4,48 | I 47,13 | N 3,44 | |

b) 5-Methoxyacetylamino-2,4,6-triiodisophthalsäure-N-(2,3-dihydroxypropyl)-monoamid

60,6 g (75,1 mmol) des unter Beispiel 9a) hergestellten Säurechlorids werden in 376 ml in einnormaler Natronlauge eingetragen und unter Stickstoffatmosphäre etwa 45 Minuten kräftig gerührt. Die Vollständigkeit des Umsatzes wird über Dünnschichtchromatographie überprüft und die Produktlösung ohne Aufarbeitung für die nächste Stufe verwendet.

c) N-Carboxymethyl-5-methoxyacetylamino-2,4,6-triiodisophthalsäure-N'-(2,3-dihydroxypropyl)-monoamid

Die nach Beispiel 9b) hergestellte Lösung des Eduktes (75,1 mmol) wird unter Stickstoffatmosphäre mit 17,5 g (150,2 mmol) des Natriumsalzes der Chloressigsäure versetzt und ca. 18 Stunden bei 90°C gerührt. Die Lösung wird mit 2 normaler Salzsäure auf pH 1 eingestellt und vollständig eingedampft. Der Rückstand wird an Kieselgel 60 (Merck) (Fließmittel Dichlormethan/Methanol/Essigsäure (2:2:1) chromatographiert. Die Produktfraktionen werden im Vakuum zur Trockne eingedampft und der Rückstand aus Methanol/Isopropanol umkristallisiert.
Ausbeute: 47,7 g (83,3 % d. Th.) farblose Kristalle

| Analyse (bezogen auf lösungsmittelfreie Substanz): | | | | | |
|---|---|---|---|---|---|
| ber. | C 25,22 | H 2,25 | I 49,96 | N 3,68 | O 18,90 |
| gef. | C 25,31 | H 2,51 | I 49,82 | N 3,72 | |

d) N-Methoxycarbonylmethyl-5-methoxyacetylamino-2,4,6-triiodisophthalsäure-N'-(2,3-dihydroxypropyl)-monoamid

45,8 g (60,1 mmol) der unter Beispiel 9c) dargestellten Verbindung wird in 150 ml wasserfreiem Methanol eingetragen und unter Stickstoffatmosphäre gerührt. Unter Rühren werden 5,6 ml (6,6 mmol) Dimethylsulfit zugetropft. Der Ansatz wird 4 Stunden bei Raumtemperatur gerührt und 1 Stunde unter Rückfluß gekocht. Anschließend wird eingedampft, der Rückstand mit Isopropanol ausgerührt, abfiltriert und im Vakuum getrocknet.
Ausbeute: 41,2 g (88,4 % d. Th.) farblose Kristalle

| Analyse (bezogen auf lösungsmittelfreie Substanz): | | | | | |
|---|---|---|---|---|---|
| ber. | C 26,31 | H 2,47 | I 49,06 | N 3,61 | O 18,55 |
| gef. | C 26,21 | H 2,35 | I 49,13 | N 3,53 | |

e) N-Methoxycarbonylmethyl-5-methoxyacetylamino-2,4,6-triiodisophthalsäure-N'-(2,3-diacetoxypropyl)-monoamid

38,2 g (49,2 mmol) der unter Beispiel 9d) dargestellten Verbindung werden in ein Gemisch aus 16,3 ml (172 mmol) Essigsäureanhydrid und 150 ml Dioxan unter Feuchtigkeitsausschluß eingerührt. Es werden 0,60 g (4,9 mmol) 4-N,N-Dimethylaminopyridin zugegeben und 2 Stunden bei 50° C gerührt. Anschließend wird der Ansatz eingedampft, der Rückstand mit Essigsäureethylester/tert.-Butylmethylether ausgerührt, abfiltriert und im Vakuum getrocknet.
Ausbeute: 37,0 g (87,4 % d.. Th.) farbloser Feststoff

| Analyse (bezogen auf lösungsmittelfreie Substanz): | | | | | |
|---|---|---|---|---|---|
| ber. | C 29,32 | H 2,70 | I 44,26 | N 3,26 | O 20,46 |

(fortgesetzt)

| Analyse (bezogen auf lösungsmittelfreie Substanz): | | | | |
|---|---|---|---|---|
| gef. | C 29,38 | H 2,77 | I 44,17 | N 3,30 |

f) N-Methoxycarbonylmethyl-5-methoxyacetylamino-2,4,6-triiodisophthalsäure-N'-(2,3-diacetoxypropyl)-amid-chlorid

35,6 g (41,4 mmol) der unter Beispiel 9e) beschriebenen Verbindung werden in 150 ml 1,2 -Dichlorethan eingetragen. Zu der unter Feuchtigkeitsausschluß bei Raumtemperatur gerührten Suspension werden 0,1 ml N,N-Dimethylformamid und 4,50 ml (62,1 mmol) Thionylchlorid gegeben. Der Ansatz wird unter Rückfluß gekocht, bis keine Gasentwicklung mehr zu beobachten ist. Die nun vorliegende Lösung wird am Vakuum eingedampft, der Rückstand in Dichlormethan aufgenommen und mit gesättigter, wäßriger Natriumhydrogencarbonatlösung ausgeschüttelt. Die organische Phase wird über wasserfreiem Magnesiumsulfat getrocknet und filtriert. Durch Zutropfen von tert.-Butylmethylether zum eingeengten Filtrat erhält man einen farblosen Niederschlag der abgesaugt und im Vakuum getrocknet wird.
Ausbeute: 30,6 g (84,1 % d. Th.) farbloser Feststoff

| Analyse (bezogen auf lösungsmittelfreie Substanz): | | | | | | |
|---|---|---|---|---|---|---|
| ber. | C 28,71 | H 2,52 | Cl 4,04 | 143,33 | N 3,19 | O 18,21 |
| gef. | C 28,81 | H 2,80 | Cl 4,28 | I 43,17 | N 3,21 | |

g) Herstellung des 3-[(N-Carboxymethyl)-methoxyacetylamino-5-(2,3-dihydroxypropylcarbamoyl)-2,4,6-triiodbenzoyl-Derivats des 48fachen primären Kaskadenamins [siehe Beispiel 1 h)]

Zu einer bei Raumtemperatur mechanisch gerührten Lösung von 21,2 g (24,1 mmol) des unter Beispiel 9f) hergestellten Säurechlorids in 100 ml N,N-Dimethylformamid wird eine Emulsion bestehend aus 3,49 g (0,34 mmol) des unter Beispiel 1h) hergestellten Kaskadenamins, 5,0 ml (36,2 mmol) Triethylamin und 30 ml Wasser langsam zugetropft. Der Ansatz wird 2 Tage bei Raumtemperatur gerührt, dann mit 60 ml 2 n Natronlauge versetzt und 2 Stunden bei 50°C gerührt. Die Lösung wird nach Abkühlen auf Raumtemperatur mit 2 n Salzsäure neutralisiert und einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 13,6 g (86,8 % d. Th.) farbloses Lyophilisat

| Analyse (bezogen auf wasserfreie Substanz): | | | | | |
|---|---|---|---|---|---|
| ber. | C 31,87 | H 3,58 | I 39,61 | N 8,50 | O 16,44 |
| gef. | C 31,98 | H 3,68 | I 39,43 | N 8,62 | |

**Beispiel 10**

Herstellung des 3-(3-Carboxypropionylamino)-5-(2,3-dihydoxypropylcarbamoyl)-2,4,6-triiodbenzoyl-Derivats des 6fachen primären Kaskadenamins [siehe Beispiel 10 a)]

a) Tris-{[7,7-bis-(4,7-diaza-3-oxoheptyl)]-4,7-diaza-3-oxoheptyl}-amin (Kaskadenamin mit 6 primären Aminogruppen)

127,0 g (121, 6 mmol) dieses Kaskadenamins werden nach der Patentschrift US 4507466 durch Umsetzung von Nitrilotripropionsäuretrimethylester (Pfaltz und Bauer) mit

1. Ethylendiamin
2. Acrylsäuremethylester,
3. Ethylendiamin hergestellt.

| Analyse (bezogen auf wasserfreie Substanz): | | | | |
|---|---|---|---|---|
| ber. | C 51,75 | H 8,98 | N 25,48, | O 13,79 |

(fortgesetzt)

| Analyse (bezogen auf wasserfreie Substanz): | | | |
|---|---|---|---|
| gef. | C 51,48 | H 9,04 | N 25,70 |

b) Herstellung des 3-(3-Carboxypropionylamino)-5-(2,3-dihydoxypropylcarbamoyl)-2,4,6-triiodbenzoyl-Derivats des 6fachen primären Kaskadenamins [siehe Beispiel 10 a)]

Zu einer bei Raumtemperatur mechanisch gerührten Lösung von 24,9 g (28,9 mmol) des unter Beispiel 1i) hergestellten Säurechlorids in 120 ml N,N-Dimethylformamid wird eine Emulsion bestehend aus 3,35 g (3,21 mmol) des unter Beispiel 1b) hergestellten Kaskadenamins, 6,01 ml (43,3 mmol) Triethylamin und 30 ml Wasser langsam zugetropft. Der Ansatz wird 2 Tage bei Raumtemperatur gerührt, dann mit 60 ml 2 n Natronlauge versetzt und 2 Stunden bei 50°C gerührt. Die Lösung wird nach Abkühlen auf Raumtemperatur mit 2 n Salzsäure neutralisiert und einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 15,0 g (76,2 % d. Th.) farbloses Lyophilisat

| Analyse (bezogen auf wasserfreie Substanz): | | | | | |
|---|---|---|---|---|---|
| ber. | C 30,43 | H 3,24 | I 42,87 | N 8,15 | O 15,31 |
| gef. | C 30,50 | H 3,24 | I 42,69 | N 8,16 | |

**Beispiel 11**

Herstellung des 3-Carboxymethylcarbamoyl-5-natriumcarboxylatomethylcarbamoyl-2,4,6-triiodphenylthiocarbamoyl-Derivats des 48fachen primären Kaskadenamins [siehe Beispiel 1h)]

a) 5-Isothiocyanato-2,4,6-triiodisophthalsäure-N,N'-bis-(methoxycarbonylmethyl)-diamid

Zu einer bei Raumtemperatur gerührten Suspension von 17,3 g (24,7 mmol) des unter Beispiel 2a) beschriebenen Anilinderivates in 170 ml 1,2-Dichlorethan werden 20 ml Polyvinylpyridin (Reillex), 50 ml Wasser und 3,66 ml (49,4 mmol) Thiophosgen in 30 ml 1,2-Dichlorethan zugegeben. Nach 3stündigem Rühren bei 50°C wird der Ansatz in Dichlormethan aufgenommen, die organische Phase wird abgetrennt, über wasserfreiem Magnesiumsulfat getrocknet und filtriert. Das Filtrat wird am Vakuum eingedampft, der Rückstand mit Essigsäureethylester ausgerührt, abgesaugt und am Vakuum getrocknet.
Ausbeute: 16,6 g (90,7 % d. Th.) hellbeiger Feststoff

| Analyse (bezogen auf lösungsmittelfreie Substanz): | | | | | | |
|---|---|---|---|---|---|---|
| ber. | C 24,25 | H 1,63 | I 51,24 | N 5,66 | O 12,92 | S 4,32 |
| gef. | C 24,33 | H 1,74 | I 51,12 | N 5,65 | | S 4,53 |

b) Herstellung des 3-Carboxymethylcarbamoyl-5-natriumcarboxylatomethylcarbamoyl-2,4,6-triiodphenylthiocarbamoyl-Derivats des 48fachen primären Kaskadenamins [siehe Beispiel 1 h)]

Zu einer bei Raumtemperatur mechanisch gerührten Lösung von 15,8 g (21,3 mmol) des unter Beispiel 11a) hergestellten Isothiocyanats in 80 ml Dimethylsulfoxid wird eine Lösung von 3,1 g (0,30 mmol) des unter Beispiel 1h) hergestellten Kaskadenamins, in 30 ml Dimethylsulfoxid zugetropft. Der Ansatz wird 2 Tage bei Raumtemperatur gerührt, dann mit 30 ml 2 n Natronlauge versetzt und 2 Stunden bei 50°C gerührt. Die Lösung wird nach Abkühlen auf Raumtemperatur mit 2 n Salzsäure neutralisiert und einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 12,1 g (88,0 % d. Th.) farbloses Lyophilisat

| Analyse (bezogen auf wasserfreie Substanz): | | | | | | | |
|---|---|---|---|---|---|---|---|
| ber. | C 28,33 | H 2,76 | I 39,91 | N 10,03 | Na 2,41 | O 13,21 | S 3,36 |

(fortgesetzt)

| Analyse (bezogen auf wasserfreie Substanz): | | | | | | |
|---|---|---|---|---|---|---|
| gef. | C 28,45 | H 2,99 | I 39,69 | N 10,20 | Na 2,19 | S 3,21 |

**Beispiel 12**

Herstellung des 3-Carboxymethylcarbamoyl-5-[(N-methoxyacetyl)-methylamino]-2,4,6-triiodbenzoyl-Derivats des 32fachen primären Kaskadenamins [siehe Beispiel 12b)]

a) N-Methoxyacetyl-5-methylamino-2,4,6-triiodisophthalsäure-N'-ethoxycarbonylmethylamid-chlorid

Eine Lösung von 68,2 g (100 mmol) N-Methoxyacetyl-5-methylamino-2,4,6-triiodisophthalsäuredichlorid (EP 0015867) in 500 ml N,N-Dimethylformamid wird mit 14,0 g (100 mmol) Glycinethylester-Hydrochlorid (Darstellung nach D.A. Hoogwater, M. Peereboom, Tetrahedron, 46, 5325-5332 (1990)) und 10,1 g (100 mmol) Triethylamin versetzt. Es entsteht eine Suspension, die über Nacht unter Argon bei Raumtemperatur gerührt wird. Anschließend wird eingedampft und der Rückstand an Kieselgel 60 (Merck) mit Dichlormethan/Essigsäureethylester chromatographiert. Nach Eindampfen der Produktfraktionen erhält man einen farblosen Feststoff, der im Vakuum getrocknet wird.
Ausbeute: 53,4 g (71,49 % d. Th.)

| Analyse (bezogen auf lösungsmittelfreie Substanz): | | | | | | |
|---|---|---|---|---|---|---|
| ber. | C 25,68 | H 2,15 | Cl 4,74 | I 50,87 | N 3,74 | O 12,83 |
| gef. | C 25,84 | H 2,31 | Cl 4,62 | I 50,59 | N 3,69 | |

b) Herstellung des 3-Carboxymethylcarbamoyl-5-[(N-methoxyacetyl)-methylamino]-2,4,6-triiodbenzoyl-Derivats des 32fachen primären Kaskadenamins [siehe Beispiel 12b)]

Zu einer bei Raumtemperatur unter Argonatmosphäre mechanisch gerührten Lösung von 31,6 g (42,2 mmol) des unter Beispiel 12a) hergestellten Säurechlorids in 200 ml N,N-Dimethylformamid werden gleichzeitig eine Lösung von 3,12 g (0,88 mmol) Kaskadenamin DAB $(PA)_4(PA)_8(PA)_{16}(PA)_{32}$ (WO 93/14147, Beispiel VIII) in 30 ml Wasser und 6,41 g (63,3 mmol) Triethylamin zugetropft. Der Ansatz wird 2 Tage bei Raumtemperatur gerührt, dann mit 50 ml 2 N Natronlauge versetzt und 1,5 Stunden bei 50°C gerührt . Die Lösung wird nach dem Abkühlen auf Raumtemperatur mit 2 N Salzsäure neutralisiert und einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 18,7 g (83,7 % d. Th.) farbloses Lyophilisat

| Analyse (bezogen auf wasserfreie Substanz): | | | | | |
|---|---|---|---|---|---|
| ber. | C 29,88 | H 3,11 | I 47,96 | N 6,95 | O 12,09 |
| gef. | C 30,04 | H 3,21 | I 47,73 | N 6,99 | |

**Beispiel 13**

Herstellung des 3-[(N-Carboxymethyl)-natriumcarboxylatomethylcarbamoyl]-5-[(Nmethoxyacetyl)-methylamino]-2,4,6-triiodbenzoyl-Derivats des 64fachen primären Kaskadenamins [siehe Beispiel 13b)]

a) N-Methoxyacetyl-5-methylamino-2,4,6-triodisophthalsäure-N,N-bis-(ethoxycarbonylmethyl)-amid-chlorid

Eine Lösung von 68,2 g (100 mmol) N-Methoxyacetyl-5-methylamino-2,4,6-triiodisophthalsäuredichlorid (EP 0015867) in 500 ml N,N-Dimethylformamid wird mit 22,6 g (100 mmol) Iminodiessigsäurediethylester-Hydrochlorid (Darstellung nach Jongkees, Recl. Trav. Chimys-Bas, 27, 296 (1908)) und 10,1 g (100 mmol) Triethylamin versetzt. Es entsteht eine Suspension, die über Nacht unter Argon bei Raumtemperatur gerührt wird. Anschließend wird eingedampft und der Rückstand an Kieselgel 60 (Merck) mit Dichlormethan/Essigsäureethylester chromatographiert. Nach Eindampfen der Produktfraktionen erhält man einen farblosen Feststoff, der im Vakuum getrocknet wird.
Ausbeute: 61,3 g (76,0 % d.Th.)

| Analyse (bezogen auf lösungsmittelfreie Substanz): | | | | | | |
|---|---|---|---|---|---|---|
| ber. | C 26,81 | H 2,25 | Cl 4,40 | I 47,21 | N 3,47 | O 15,87 |
| gef. | C 26,79 | H 2,32 | Cl 4,43 | I 47,15 | N 3,52 | |

b) Herstellung des 3-[(N-Carboxymethyl)-natriumcarboxylatomethylcarbamoyl]-5-[(Nmethoxyacetyl)-methylamino]-2,4,6-triiodbenzoyl-Derivats des 64fachen primären Kaskadenamins [siehe Beispiel 13b)]

Zu einer bei Raumtemperatur unter Argonatmosphäre mechanisch gerührten Lösung von 59,8 g (74,1 mmol) des unter Beispiel 13a) hergestellten Säurechlorids in 200 ml N,N-Dimethylformamid werden gleichzeitig eine Lösung von 5,54 g (0,77 mmol) Kaskadenamin DAB $(PA)_4(PA)_8(PA)_{16}(PA)_{32}(PA)_{64}$ (WO 93/14147, Beispiel X) in 50 ml Wasser und 15,4 ml (111 mmol) Triethylamin zugetropft. Der Ansatz wird 2 Tage bei Raumtemperatur gerührt, dann mit 100 ml 2 N Natronlauge versetzt und 2 Stunden bei 50°C gerührt . Die Lösung wird nach dem Abkühlen auf Raumtemperatur mit 2 N Salzsäure neutralisiert, und einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 40,4 g (93,2 % d. Th.) farbloses Lyophilisat

| Analyse (bezogen auf wasserfreie Substanz): | | | | | | |
|---|---|---|---|---|---|---|
| ber. | C 29,99 | H 2,96 | I 43,46 | N 6,35 | O 14,61 | Na 2,62 |
| gef. | C 30,27 | H 3,19 | I 43,18 | N 6,48 | | Na 2,36 |

**Beispiel 14**

Herstellung des 2-{3-[(N-carboxymethyl)-methylcarbamoyl]-5-[(N-natriumcarboxylatomethyl)-methylcarbamoyl]-2,4,6-triiodphenylcarbamoyl}-ethyl-Derivats des 24fachen primären Kaskadenamins [siehe Beispiel 1 f)]

a) 5-Acrylamido-2,4,6-triiodisophtalsäure-N,N'-bis-(ethoxycarbonyl)-N,N'-dimethyldiamid

8,7 g des unter Beispiel 4a) hergestellten Anilinderivates in 45 ml N,N-Dimethylacetamid werden bei 0°C mit 3,12 g (34,5 mmol) destilliertem Acrylsäurechlorid versetzt. Man rührt die Reaktionsmischung für 18 Stunden bei Raumtemperatur und gießt anschließend in Eiswasser. Der entstandene Niederschlag wird abgesaugt und mit Wasser neutral gewaschen. Man trocknet das Rohprodukt im Vakuum bei 50°C und reinigt es über eine HPLC (stationäre Phase: RP18, mobile Phase: Wasser/Acetonitril). Nach dem Eindampfen der Produktfraktionen wird der Rückstand im Hochvakuum aufgeschäumt.
Ausbeute: 7,4 g (79 % d. Th.) farbloser Feststoff.

| Analyse (bezogen auf wasserfreie Substanz): | | | | | |
|---|---|---|---|---|---|
| ber. | C 31,09 | H 2,98 | I 46,94 | N 5,18 | O 13,81 |
| gef. | C 30,96 | H 3,18 | I 46,72 | N 5,04 | |

b) Herstellung des 2-{3-[(N-carboxymethyl)-methylcarbamoyl]-5-[(N-natriumcarboxylatomethyl)-methylcarbamoyl]-2,4,6-triiodphenylcarbamoyl}-ethyl-Derivats des 24fachen primären Kaskadenamins [siehe Beispiel 1 f)]

Zu einer Lösung von 7,05 g (8,7 mmol) Acrylamid aus Beispiel 14a) in 25 ml N,N-Dimethylformamid werden bei Raumtemperatur 0,895 g (0,18 mmol) Polyamin (Beispiel 1f) addiert und die Reaktionsmischung bei 75°C für 5 Stunden gerührt. Nach abgeschlossener Umsetzung wird der Ansatz im Vakuum zur Trockne eingedampft und für 2 Stunden bei 50 °C mit zweinormaler Natronlauge versetzt. Wenn die Verseifung erfolgt ist, wird die Lösung neutralisiert und zur Abtrennung niedermolekularer Bestandteile über eine Hohlfasermembran (H1 P3-20, Amicon) ultrafiltriert. Die wäßrige Produktlösung wird über ein Cellulose-Membranfilter (Porengröße 0,45 µm) filtriert und gefriergetrocknet.
Ausbeute: 6,85 g (92 % d. Th.) farbloses Lyophilisat.

| Analyse (bezogen auf wasserfreie Substanz): | | | | | | |
|---|---|---|---|---|---|---|
| ber. | C 30,03 | H 1,83 | I 44,28 | N 7,87 | O 14,65 | Na 1,34 |

(fortgesetzt)

| Analyse (bezogen auf wasserfreie Substanz): | | | | | |
|---|---|---|---|---|---|
| gef. | C 29,87 | H 1,97 | I 44,19 | N 7,61 | Na 1,16 |

**Beispiel 15**

Herstellung des N-Methoxyacetyl-3-methylamino-5-(2-sulfoethylcarbamoyl)-2,4,6-triiodbenzoyl-Derivats des 48fachen primären Kaskadenamins [siehe Beispiel 1 h)]

a) N-Methoxyacetyl-5-methylamino-2,4,6-triiodisophthalsäure-N'-(2-bromethyl)-amidchlorid

Eine Lösung von 68,2 g (100 mmol) N-Methoxyacetyl-5-methylamino-2,4,6-triiodisophthalsäuredichlorid (EP 0015867) in 500 ml N,N-Dimethylformamid wird mit 20,5 g (100 mmol) 2-Bromethylamin-Hydrobromid und 20,2 g (200 mmol) Triethylamin versetzt. Es entsteht eine Suspension, die über Nacht unter Argon bei Raumtemperatur gerührt wird. Anschließend wird eingedampft und der Rückstand an Kieselgel 60 (Merck) mit Dichlormethan/Essigsäureethylester chromatographiert. Nach Eindampfen der Produktfraktionen erhält man einen farblosen Feststoff, der im Vakuum getrocknet wird.
Ausbeute: 55,3 g (71,88 % d. Th.)

| Analyse (bezogen auf lösungsmittelfreie Substanz): | | | | | | | |
|---|---|---|---|---|---|---|---|
| ber. | C 21,86 | H 1,70 | Br 10,39 | Cl 4,61 | I 49,49 | N 3,64 | O 8,32 |
| gef. | C 21,84 | H 1,81 | Br 10,48 | Cl 4,62 | I 49,59 | N 3,69 | |

b) Herstellung des N-Methoxyacetyl-3-methylamino-5-(2-bromethylcarbamoyl)-2,4,6-triiodbenzoyl-Derivats des 48fachen primären Kaskadenamins [siehe Beispiel 1 h)]

Zu einer bei Raumtemperatur unter Argonatmosphäre mechanisch gerührten Lösung von 53,8 g (69,9 mmol) des unter Beispiel 15a) hergestellten Säurechlorids in 200 ml N,N-Dimethylformamid werden gleichzeitig eine Lösung von 10,1 g (0,97 mmol) des in Beispiel 1 h) beschriebenen Kaskadenamins in 50 ml Wasser und 12,6 ml (90,9 mmol) Triethylamin zugetropft. Der Ansatz wird 2 Tage bei Raumtemperatur gerührt, dann mit 2 N Salzsäure neutralisiert, und einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 39,5 g (89,2 % d. Th.) farbloses Lyophilisat

| Analyse (bezogen auf wasserfreie Substanz): | | | | | | |
|---|---|---|---|---|---|---|
| ber. | C 29,71 | H 3,30 | Br 8,41 | I 40,08 | N 8,60 | O 9,90 |
| gef. | C 29,83 | H 3,37 | Br 8,52 | I 39,90 | N 8,55 | |

c) Herstellung des N-Methoxyacetyl-3-methylamino-5-(2-sulfoethylcarbamoyl)-2,4,6-triiodbenzoyl-Derivats des 48fachen primären Kaskadenamins [siehe Beispiel 1 h)]

37,4 g (0,82 mmol) der in Beispiel 15 b) beschriebenen Verbindung werden in 500 ml Wasser mit 49,6 g (393 mmol) Natriumsulfit versetzt und 72 Stunden bei 25°C gerührt. Anschließend wird die wäßrige Lösung einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 31,2 g (83,2 % d. Th.) farbloses Lyophilisat

| Analyse (bezogen auf wasserfreie Substanz): | | | | | | |
|---|---|---|---|---|---|---|
| ber. | C 29,66 | H 3,40 | I 40,00 | N 8,58 | O 14,99 | S 3,37 |
| gef. | C 29,73 | H 3,47 | I 39,91 | N 8,65 | | S 3,22 |

**Beispiel 16**

Herstellung des 3,5-Di-(natriumphosphonomethylcarbamoyl)-2,4,6-triiodarylcarbamoyl-Derivats des 12fachen primären Kaskadenamins [siehe Beispiel 1 d)]

a) 5-Amino-2,4,6-triiodisophtalsäure-N,N'-bis-(diethylphosphonomethyl)-diamid

Eine Lösung von 59,6 g (100 mmol) 5-Amino-2,4,6-triiodisophthalsäuredichlorid (DOS 29 26 428) in 300 ml N,N-Dimethylformamid wird mit 36,8 g (220 mmol) Aminomethanphosphonsäurediethylester und 61,0 ml (440 mmol) Triethylamin versetzt. Es entsteht eine Suspension, die über Nacht bei Raumtemperatur unter Argon gerührt wird. Nach dem Eindampfen der Suspension im Vakuum wird der Rückstand aus Methanol umkristallisiert.
Ausbeute: 59,8 g (69,8 % d. Th.)

| Analyse (bezogen auf lösungsmittelfreie Substanz): | | | | | | |
|---|---|---|---|---|---|---|
| ber. | C 25,22 | H 3,29 | I 44,42 | N 4,90 | P 7,23 | O 14,93 |
| gef. | C 25,16 | H 3,41 | I 44,26 | N 4,78 | P 7,17 | |

b) 5-Isocyanato-2,4,6-triiodisophthalsäure-N,N'-bis-(diethylphosphonomethyl)-diamid

Zu einer bei 65°C Ölbadtemperatur unter Argonatmosphäre gerührten Suspension von 25,7 g (30 mmol) des unter Beispiel 16a) hergestellten Anilinderivates in 200 ml 1,2 -Dichlorethan werden 147 ml (73,8 mmol) einer zweinormalen toluolischen Phosgenlösung und 2 ml N,N-Dimethylformamid zugegeben. Nach vollständiger Umsetzung des Anilinderivates wird der Ansatz im Vakuum eingedampft, der Rückstand mit wasserfreiem Essigsäureethylester ausgerührt, unter Stickstoffatmosphäre abgesaugt und im Ölpumpenvakuum getrocknet.
Ausbeute: 25,6 g (96,7 % d. Th.) hellbeiger Feststoff

| Analyse (bezogen auf lösungsmittelfreie Substanz): | | | | | | |
|---|---|---|---|---|---|---|
| ber. | C 25,84 | H 2,97 | I 43,11 | N 4,76 | P 7,02 | O 16,31 |
| gef. | C 25,72 | H 3,08 | I 42,98 | N 4,59 | P 6,91 | |

c) Herstellung des 3,5-Di-(natriumphosphonomethylcarbamoyl)-2,4,6-triiodarylcarbamoyl-Derivats des 12fachen primären Kaskadenamins [siehe Beispiel 1 d)]

Zu einer bei Raumtemperatur unter Argonatmosphäre gerührten Lösung von 22,5 g (25,5 mmol) des unter Beispiel 16b) hergestellten Isocyanats in 220 ml wasserfreiem Dimethylsulfoxid wird eine Lösung von 4,68 g (2,12 mmol) des unter Beispiel 1d) hergestellten Kaskadenamins in 47 ml wasserfreiem Dimethylsulfoxid langsam zugetropft. Der Ansatz wird 3 Tage bei Raumtemperatur gerührt, dann im Hochvakuum eingedampft, mit 14,11 ml (110,5 mmol) Bromtrimethylsilan versetzt und für 26 Stunden bei 45°C gerührt. Das Rohprodukt wird tropfenweise mit 150 ml Wasser versetzt und 4 Stunden bei Raumtemperatur gerührt. Nach Neutralisation mit Natronlauge wird die Produktlösung einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 19,98 g (78,7 % d. Th.) gelbliches Lyophilisat.

| Analyse (bezogen auf wasserfreie Substanz): | | | | | | | |
|---|---|---|---|---|---|---|---|
| ber. | C 22,86 | H 2,47 | I 38,14 | N 8,89 | Na 4,61 | P 6,21 | O 16,83 |
| gef. | C 22,69 | H 2,61 | I 38,03 | N 8,65 | Na 4,49 | P 6,08 | |

**Beispiel 17**

Herstellung des Triacontakis-{3-(4-Carboxy-3-oxabutyrylamino)-5-(2,3-dihydroxypropylcarbamoyl)-2,4,6-triiodbenzoyl}-bis-(4-carboxy-3-oxabutyryl)-Derivats des 32fachen primären Kaskadenamins [siehe Beispiel 12]

Zu einer Raumtemperatur unter Argonatmosphäre mechanisch gerührten Lösung von 44,6 g (50,0 mmol) des unter Beispiel 6b) hergestellten Säurechlorids in 250 ml N,N-Dimethylformamid werden gleichzeitig eine Lösung

von 5,53 g (1,56 mmol) Kaskadenamin DAB $(PA)_4(PA)_8(PA)_{16}(PA)_{32}$ [WO 93/14147, Beispiel VIII] in 40 ml Wasser und 13,9 ml (100 mmol) Triethylamin zugetropft. Der Ansatz wird 2 Tage bei Raumtemperatur gerührt, dann mit 1,16 g (10,0 mmol) Diglykolsäureanhydrid versetzt und weitere 24 Stunden gerührt. Anschließend gibt man 50 ml 2 N Natronlauge zu und rührt 1,5 Stunden bei 50 °C. Die Lösung wird nach dem Abkühlen auf Raumtemperatur mit 2 N Salzsäure neutralisiert und einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 35,6 g (88,8 % d.Th.) farbloses Lyophilisat

| Analyse (bezogen auf wasserfreie Substanz): | | | | | |
|---|---|---|---|---|---|
| ber. | C 30,03 | H 3,26 | I 44,49 | N 6,76 | O 15,45 |
| gef. | C 30,14 | H 3,21 | I 44,43 | N 6,69 | |

**Beispiel 18**

Herstellung des 3-(4-Carboxy-3-oxabutyrylamino)-5-(2,3,4,5,6-pentahydroxyhexylcarbamoyl)-2,4,6-triiodbenzoyl-Derivats des 48-fachen primären Kaskadenamins [siehe Beispiel 1 h)]

a) 3-Amino-5-(2,3,4,5,6-pentaacetoxyhexylcarbamoyl)-2,4,6-triiodbenzoesäure

14,7 g (20 mmol) 3-Amino-5-(2,3,4,5,6-pentahydroxyhexylcarbamoyl)-2,4,6-triiodbenzoesäure (DOS 1928838) werden in 30 ml N,N-Dimethylacetamid gelöst und mit 25 mg 4-(Dimethylamino)-pyridin bei Raumtemperatur versetzt. Man kühlt auf 0 °C und gibt innerhalb von 30 Minuten tropfenweise 11,3 ml (120 mmol) Acetanhydrid hinzu. Nach weiteren 30 Minuten bei dieser Temperatur läßt man über Nacht auf Raumtemperatur kommen. Das überschüssige Acetanhydrid wird mit Methanol umgesetzt und die Reaktionsmischung eingedampft. Der Rückstand wird in 100 ml Butylacetat aufgenommen und sukzessive mit Natriumhydrogencarbonatlösung und gesättigter Natriumchloridlösung gewaschen. Nach dem Trocknen der organischen Phase über Natriumsulfat wird die Lösung zur Trockne eingedampft. Das Rohprodukt kann ohne weitere Reinigung in die nächste Stufe eingesetzt werden.
Ausbeute: 16,2 g (85,6 % d. Th.) gelblicher Schaum.

b) 3-Amino-5-(2,3,4,5,6-pentaacetoxyhexylcarbamoyl)-2,4,6-triiodbenzoesäurechlorid

15 5 g (16,4 mmol) 3-Amino-5-(2,3,4,5,6-pentaacetoxyhexylcarbamoyl)-2,4,6-triiodbenzoesäure [Beispiel 18 a)] werden in 80 ml Essigsäureethylester suspendiert und mit 1,9 ml (24,6 mmol) Thionylchlorid für 5 Stunden am Rückfluß erhitzt. Die Reaktionsmischung gibt man zu 30 g Natriumhydrogencarbonat in 300 ml Wasser und rührt 1,5 Stunden kräftig. Anschließend trennt man die Phasen und trocknet die organische Phase über Natriumsulfat, filtriert und dampft die Lösung ein. Das ölige Reaktionsprodukt wird im Hochvakuum aufgeschäumt.
Ausbeute: 14,9 g (94 % d. Th.) hellgelber Schaum.

| Analyse (bezogen auf lösungsmittelfreie Substanz): | | | | | | |
|---|---|---|---|---|---|---|
| ber. | C 31,13 | H 2,93 | Cl 3,68 | I 39,47 | N 2,90 | O 19,90 |
| gef. | C 31,35 | H 3,06 | Cl 3,52 | I 39,28 | N 2,74 | |

c) 5-[4-(2-Methylethyloxycarbonyl)-3-oxabutyrylamino]-5-(2,3,4,5,6-pentaacetoxyhexylcarbamoyl)-2,4,6-triiodbenzoesäurechlorid

Zu einer unter Feuchtigkeitsausschluß gerührten Suspension von 12,9 g (13,3 mmol) 3-Amino-5-(2,3,4,5,6-pentaacetoxyhexylcarbamoyl)-2,4,6-triiodbenzoesäurechlorid [Beispiel 18 b)] in 50 ml wasserfreiem Dioxan werden 3,9 g (20 mmol) das nach Beispiel 6a) hergestellten Säurechlorids bei Raumtemperatur zugegeben. Der Ansatz wird 6 Stunden am Rückfluß gekocht, bis laut Dünnschichtchromatographie kein Edukt mehr nachweisbar ist, dann wird eingedampft, der Rückstand in Dichlormethan aufgenommen und mit gesättigter, wäßriger Natriumhydrogencarbonatlösung ausgeschüttelt. Die organische Phase wird nach Trocknen über wasserfreiem Magnesiumsulfat eingedampft und der Rückstand aus Essigsäureethylester/tert.-Butylmethylether umkristallisiert.
Ausbeute: 11,3 g (75,7 % d. Th.) farblose Kristalle.

| Analyse (bezogen auf lösungsmittelfreie Substanz): | | | | | | |
|---|---|---|---|---|---|---|
| ber. | C 34,23 | H 3,41 | Cl 3,16 | I 33,91 | N 2,50 | O 22,80 |
| gef. | C 34,45 | H 3,53 | Cl 3,12 | I 33,80 | N 2,34 | |

d) 3-(4-Carboxy-3-oxabutyrylamino)-5-(2,3,4,5,6-pentahydroxyhexylcarbamoyl)-2,4,6-triiodbenzoyl-Derivat des 48-fachen primären Kaskadenamins [siehe Beispiel 1 h)]

Zu einer bei Raumtemperatur mechanisch gerührten Lösung von 10,8 g (9,6 mmol) des unter Beispiel 18c) hergestellten Säurechlorids in 40 ml N,N-Dimethylformamid wird eine Emulsion bestehend aus 1,4 g (0,13 mmol) des unter Beispiel 1h) hergestellten Kaskadenamins, 2,0 ml (15,0 mmol) Triethylamin und 10 ml Wasser langsam zugetropft. Der Ansatz wird 2 Tage bei Raumtemperatur gerührt, dann mit 20 ml 2 n Natronlauge versetzt und 2 Stunden bei 50 °C gerührt. Die Lösung wird nach Abkühlen auf Raumtemperatur mit 2 n Salzsäure neutralisiert und dann einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.
Ausbeute: 5,4 g (82,3 % d.Th.) farbloses Lyophilisat.

| Analyse (bezogen auf wasserfreie Substanz): | | | | | |
|---|---|---|---|---|---|
| ber. | C 32,57 | H 3,85 | I 36,22 | N 7,77 | O 19,60 |
| gef. | C 32,34 | H 3,97 | I 36,13 | N 7,52 | |

**Beispiel 19**

Herstellung des 3-(2,3-Dihydroxy-3-carboxypropionylamino)-5-(2,3-dihydroxypropylcarbamoyl)-2,4,6-triiodbenzoyl-Derivats des 48-fachen primären Kaskadenamins [siehe Beispiel 1 h)]

a) 5-(2,3-Diacetoxy-3-methoxycarbonylpropionylamino)-2,4,6-tdiodisophthalsäure-N-(2,3-diacetoxypropyl)-amid-chlorid

Zu einer unter Feuchtigkeitsausschluß gerührten Suspension von 25,37 g (35,43 mmol) 5-Amino-2,4,6-triiodisophthalsäure-N-(2,3-diacetoxypropyl)-amid (EP 0308364) in 150 ml Essigsäureethylester werden 17,58 g (70,87 mmol) O,O-Diacetylweinsäuremonomethylester und 7,71 ml (106,3 mmol) Thionylchlorid bei Raumtemperatur zugegeben. Der Ansatz wird mehrere Stunden am Rückfluß gekocht, bis die Umsetzung laut Dünnschichtchromatographie vollständig ist ist; dann wird eingedampft, der Rückstand in Dichlormethan aufgenommen und mit gesättigter wäßriger Natriumhydrogencarbonatlösung ausgeschüttelt. Die organische Phase wird nach Trocknen über wasserfreiem Magnesiumsulfat eingedampft und der Rückstand an Kieselgel mit Dichlormethan/Essigsäureethylester chromatographiert.
Ausbeute: 25,57 g (74,81 % d. Th.)

| Analyse (bezogen auf losungsmittelfreie Substanz): | | | | | | |
|---|---|---|---|---|---|---|
| ber. | C 29,88 | H 2,51 | Cl 3,68 | I 39,47 | N 2,90 | O 21,56 |
| gef. | C 29,94 | H 2,63 | Cl 3,81 | I 39,30 | N 2,82 | |

b) 3-(2,3-Dihydroxy-3-carboxypropionylamino)-5-(2,3-dihydroxypropylcarbamoyl)-2,4,6-triiodbenzoyl-Derivats des 48-fachen primären Kaskadenamins [siehe Beispiel 1 h)]

Zu einer bei Raumtemperatur mechanisch gerührten Lösung von 25,6 g 26,5 mmol) des unter Beispiel 19a) hergestellten Säurechlorids in 100 ml N,N-Dimethylformamid wird eine Emulsion, bestehend aus 3,84 g (0,37 mmol) des unter Beispiel 1h) hergestellten Kaskadenamins, 5,51 ml (39,8 mmol) Triethylamin und 30 ml Wasser langsam zugetropft. Der Ansatz wird 2 Tage bei Raumtemperatur gerührt, anschließend mit 50 ml 2 n Natronlauge versetzt und 2 Stunden bei 50 °C gerührt; nach Abkühlen auf Raumtemperatur wird die Lösung mit 2 n Salzsäure neutralisiert und einer Ultrafiltration unterzogen, wobei niedermolekulare Bestandteile durch eine Hohlfasermembran (H1 P3-20, Amicon) abgetrennt werden. Die wäßrige Produktlösung wird über ein Cellulose-Membranfilter (Porengröße 0,45 µm, Sartorius) filtriert und gefriergetrocknet.

Ausbeute: 14,7 g (86,3 % d. Th.) farbloses Lyophilisat

| Analyse (bezogen auf wasserfreie Substanz): | | | | | |
|---|---|---|---|---|---|
| ber. | C 30,56 | H 3,36 | I 39,53 | N 8,48 | O 18,07 |
| gef. | C 30,69 | H 3,42 | I 39,40 | N 8,55 | |

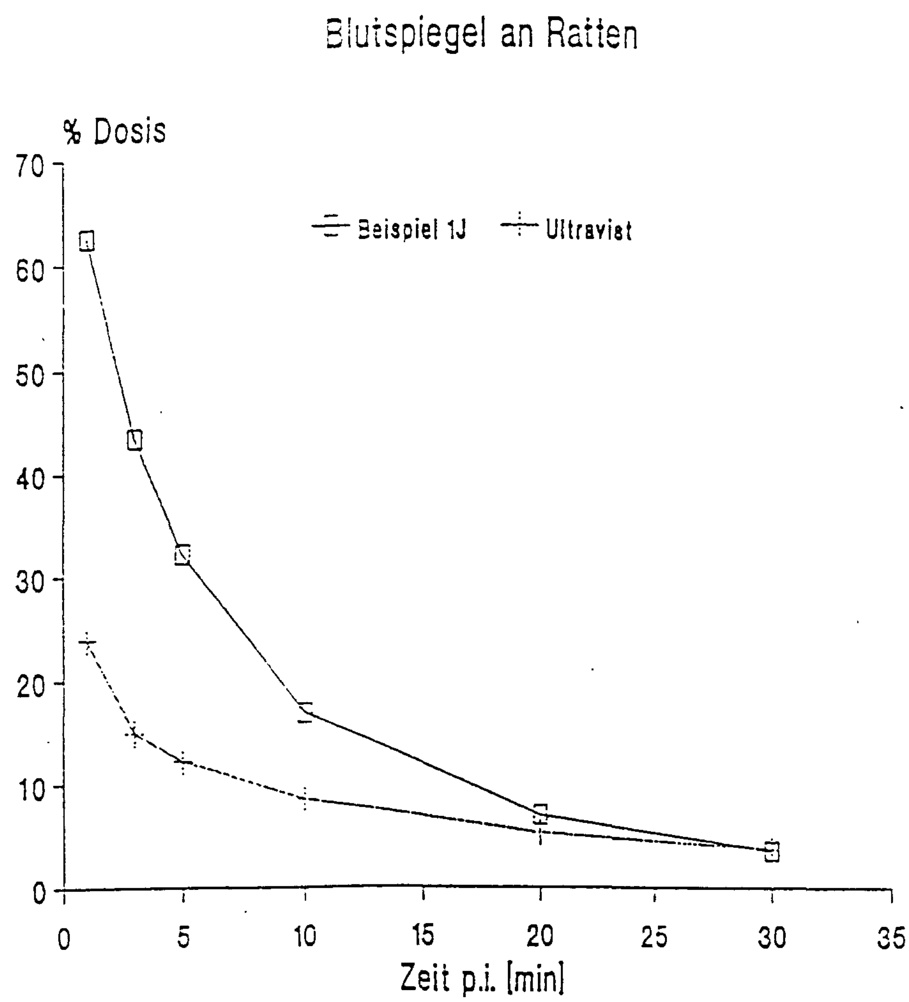


Blutspiegel bei der Ratte nach einmaliger intravenöser Injektion von 300 mg I/kg Körpergewicht von Ultravist® und der Verbindung nach Beispiel 1j. Die Daten repräsentieren den Mittelwert aus je vier Tieren.

Trotz Applikation der gleichen Dosis fallt die Blutkonzentration von Ultravist ® sehrviel schneller ab als die Konzentration des Polymers nach Beispiel 1j. Dies läßt sich auf die rasche Verteilung von Ultravist® vom Blutraum in den interstitiellen Raum zurückführen, wohingegen die Verteilung des Polymers nach Beispiel 1j auf den Blutraum beschränkt ist.

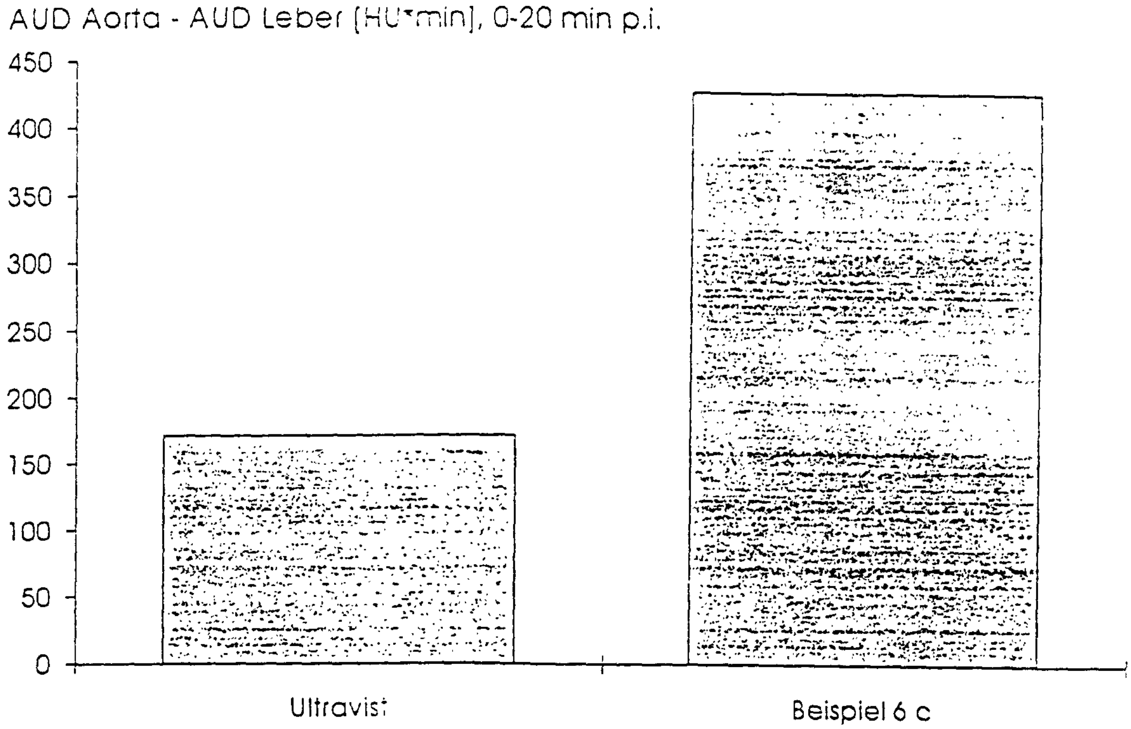


Sowohl das monomere Röntgenkontrastmittel Ultravist® (Iopromid) als auch die Verbindung Beispiel 6c (Makromolekül) wurden mit einer Dosierung von 200 mg I/kg intravenös im Bolus am Kaninchen (2 kg, Weisser Neuseeländer) appliziert (n=1 pro Substanz). Von 0 bis 20 Minuten nach Applikation wurde der Signalanstieg in Hounsfield Units (HU) im Leberparenchym und in der Aorta gemessen. Dazu wurde ein Spiral-CT (Somatom plus) der Firma Siemens verwendet. Die Aufnahmen wurden bei 120 kV durchgeführt. Es wurde für beide Substanzen die Signal-Zelt-Kurve in der Aorta und in der Leber gemessen. Die Fläche unter den Kurven wurde berechnet (AUD = area under the data). Als Maß für die Kontrastqualität der Substanzen wurde der Signalunterschied zwischen Aorta und Leberparenchym über den Zeitraum 0-20 min verwendet (AUD Aorta - AUD Leber).
Die Abbildung zeigt deutlich, daß der Signalunterschied zwischen dem Blutgefäß (Aorta) und dem umliegenden Lebergewebe für die Verbindung Beispiel 6 c (Makromolekül) deutlich höher ist als bei dem Monomeren Ultravist.

**Patentansprüche**

**1.** Iodhaltige dendrimere Polymere der allgemeinen Formel I

$$A\text{-}(X)_b \qquad \text{(I),}$$

worin

A für einen stickstoffhaltigen Kern der Basismultiplizität b steht, wobei
b für die Ziffern 1 bis 8 steht und
X für einen aus

$$\sum_{k=0}^{n-1} 2^k$$

Reproduktionseinheiten S und maximal $2^n$ bildgebenden Resten Z zusammengesetzten Rest steht, worin

n die Anzahl der Generationen bestimmt und für die Ziffern 1 bis 10 steht,

S für einen Rest der Formel II steht

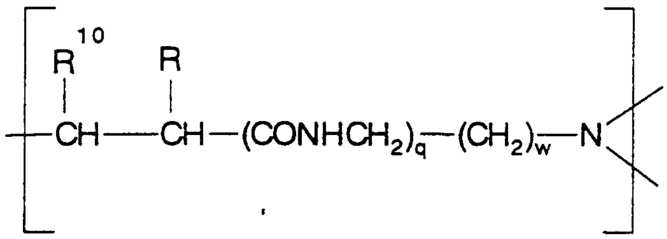

(II),

worin

| | |
|---|---|
| R und $R^{10}$ | unabhängig voneinander für ein Wasserstoffatom oder eine Methylgruppe, |
| w | für die Ziffern 1 oder 2, |
| q | für die Ziffern 0 oder 1 steht und die Positionen |
| α | für $0 \le k \le n-1$ durch weitere Reproduktionseinheiten S besetzt werden und für die n-te Generation durch die Reste Z besetzt sind, wobei gegebenenfalls nicht durch Z besetzte Positionen α durch die Reste $-(CO)_q$-U-COOH besetzt sind, worin q die oben angegebene Bedeutung hat und U für eine direkte Bindung oder eine Alkylenkette mit bis zu 6 C-Atomen, die gegebenenfalls durch 1 - 2 Sauerstoffatome unterbrochen und/oder gegebenenfalls durch 1 - 4 Hydroxygruppen und/oder durch 1-2 Carboxygruppen substituiert ist, stehen, mit der Maßgabe, daß maximal 20 % der Positionen α durch diesen Rest $-(CO)_q$-U-COOH besetzt sind, |
| Z | aus einem Verknüpfungsglied Y und einem Triiodaromaten B bestehenden bildgebenden Rest Y-B be steht, wobei Y oder B mindestens eine aliphatische Carboxy-, eine Sulfo- oder Phosphonogruppe enthalten und |
| Y | für eine Gruppe -CO-, -CONH- -CSNH-, |

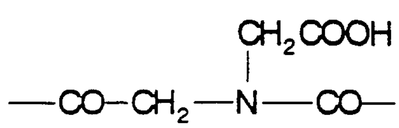
,
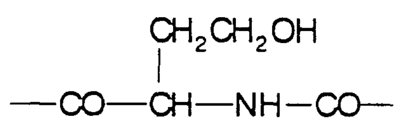
,

-CHR-CHR-CONH-

oder

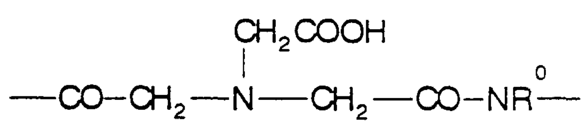

mit R in der oben genannten Bedeutung steht und $R^0$ in der Bedeutung eines Wasserstoffatoms, einer Methyl- oder einer Carboxymethylgruppe und

B für einen Benzolring

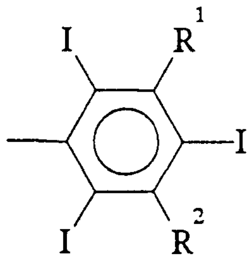

steht,
worin
$R^1$ und $R^2$ jeweils unabhängig voneinander ein Wasserstoffatom, eine $-CONR^3R^4-$ oder $-NR^6COR^5$-Gruppe bedeuten,
wobei

| | |
|---|---|
| $R^3$ und $R^4$ | unabhängig voneinander für ein Wasserstoffatom, eine gegebenenfalls durch 1 - 5 Hydroxy- und/oder 1 - 3 $C_1$-$C_3$-Alkoxyund/oder 1-3 Carboxy-, Sulfo- oder Phosphonogruppe(n) substituierte gerad- oder verzweigtkettige oder cyclische Alkylgruppe mit bis zu 12 C-Atomen, |
| $R^3$ und $R^4$ | gemeinsam mit dem Stickstoffatom für einen gegebenenfalls ein Sauerstoffatom, eine $SO_2$-Gruppe oder einen $N-CO-R^7$-Rest - mit $R^7$ in der Bedeutung einer Carboxygruppe oder einer gegebenenfalls 1 - 5 Hydroxy-, 1-3 $C_1$-$C_3$-Alkoxy- oder 1-3 Carboxy-, Sulfo- oder Phosphonogruppe(n) enthaltenden Alkylgruppe mit bis zu12 C-Atomen - enthaltenden 5- oder 6-Ring, |
| $R^5$ | für eine Carboxygruppe, eine gegebenenfalls durch ein Sauerstoffatom unterbrochene und/oder gegebenenfalls durch 1 - 3 Carboxy-, Sulfo- oder Phosphono- und/oder 1 - 5 Hydroxyund/oder 1-3 $C_1$-$C_3$-Alkoxygruppe(n) substituierte Alkylgruppe mit bis zu 12 C-Atomen, |
| $R^6$ | für ein Wasserstoffatom, eine gegebenenfalls durch 1 - 3 Carboxy-, Sulfo- oder Phosphonogruppe(n) und/oder gegebenenfalls durch 1 - 3 Hydroxygruppe(n) und/oder 1 - 3 $C_1$-$C_3$-Alkoxygruppe(n) substituierte Alkylgruppe mit bis zu 12 C-Atomen |

stehen, wobei die Reproduktionseinheiten S nur für eine Generation identisch sein müssen,
sowie deren Salze mit physiologisch unbedenklichen organischen und/oder anorganischen Basen, Aminosäuren oder Aminosäureamiden.

**2.** Iodhaltige dendrimere Polymere gemäß Anspruch 1, **dadurch gekennzeichnet, daß** der Kern A für ein Stickstoffatom, einen Rest $\beta-NR^8-\beta$, $\beta-NR^8R^9$ oder einen Rest der allgemeinen Formeln III, IV, V oder VI steht,

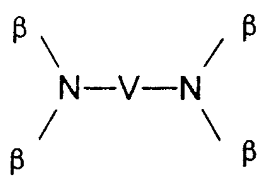

(III),

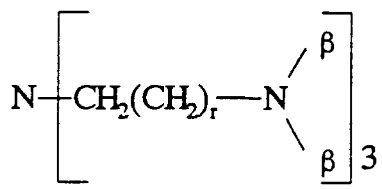

(IV),

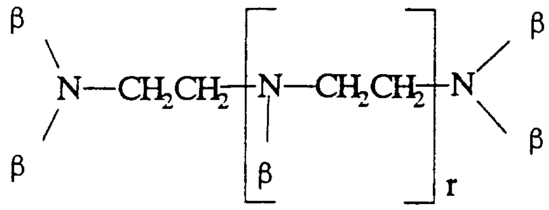

(V),

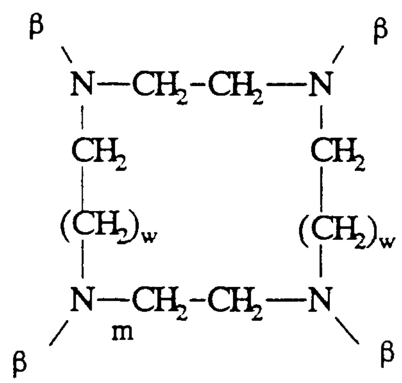

(VI),

worin

| | |
|---|---|
| $R^8$ und $R^9$ | unabhängig voneinander für einen geradkettigen oder verzweigten Alkyl-, Aryl- oder Aralkylrest mit bis zu 20 C-Atomen, der gegebenenfalls durch 1 - 4 Hydroxygruppe(n) substituiert ist, |
| β | die Bindungsstelle zum Rest X markiert, wobei die Anzahl der β mit der Basismultiplizität b gleichzusetzen ist, |
| V | für einen geradkettigen oder verzweigten Alkylen-, Arylen- oder Aralkylenrest mit bis zu 20 C-Atomen steht, der gegebenenfalls durch 1 - 4 Sauerstoffatom(e) unterbrochen und/oder durch 1 - 4 Hydroxygruppe(n) substituiert ist, |
| r | für die Ziffern 1, 2 oder 3, |
| w | für die Ziffern 1 oder 2 und |
| m | für die Ziffern 0, 1, 2 oder 3 steht. |

**3.** Iodhaltige dendrimere Polymere gemäß Anspruch 1 und 2, **dadurch gekennzeichnet, daß** der Kern A für eine Gruppe $\beta_2N\text{-}(CH_2)_4\text{-}N\beta_2$, $\beta_2N(CH_2)_2N\beta_2$, $\beta N(CH_2CH_2N\beta_2)_2$, $N(CH_2CH_2N\beta_2)_3$,

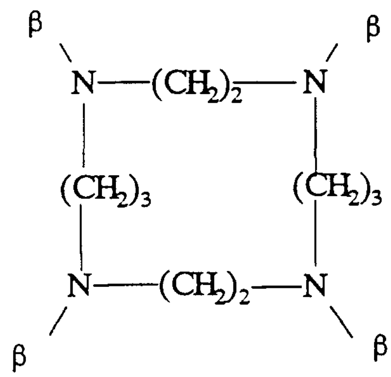

oder für

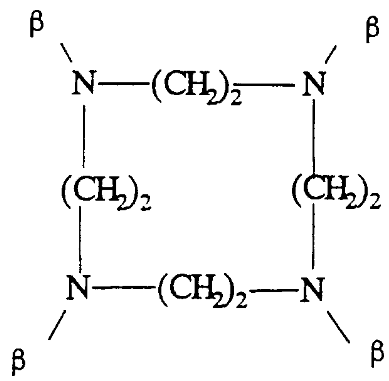

steht.

**4.** Iodhaltige dendrimere Polymere gemäß Anspruch 1 und 2, **dadurch gekennzeichnet, daß** S für

$$-CH(CH_3)CH(CH_3)CONH\text{-}CH_2\text{-}CH_2\text{-}N\ ;$$

$$-CH(CH_3)CH(CH_3)CH_2N\ ;$$

$$-CH_2CH_2CH_2\text{-}N\ ;$$

$$-CH_2CH(CH_3)CH_2\text{-}N\ ;$$

$$-CH_2CH_2\text{-}CONH\text{-}CH_2CH_2\text{-}N\ ;$$

$$-CH(CH_3)CH_2CH_2N\ ;$$

$$-CH(CH_3)CH_2CONH\text{-}CH_2CH_2\text{-}N\ ;$$

$$-CH_2CH(CH_3)CONH-CH_2-CH_2-N\ .$$

steht.

**5.** Iodhaltige dendrimere Polymere gemäß Anspruch 1 und 2, **dadurch gekennzeichnet, daß** n für die Ziffern 2-6 steht.

**6.** Iodhaltige dendrimere Polymere gemäß Anspruch 1 und 2, **dadurch gekennzeichnet, daß** $R^1$ bzw. $R^2$ für eine Gruppe $CONH_2$, $CONHCH_2COOH$, $CON(CH_2COOH)_2$, $CONHCH_2CH(OH)CH_2OH$, $CON(CH_3)CH_2COOH$, $CONHCH_2PO_3H_2$, $CON(CH_2PO_3H_2)_2$, $CON(CH_2COOH)CH_2PO_3H_2$, $CON(CH_3)CH_2CH(OH)CH_2OH$, $CONHCH_2CH_2SO_3H$, $CON(CH_2CH_2SO_3H)_2$
steht.

**7.** Iodhaltige dendrimere Polymere gemäß Anspruch 1 und 2, **dadurch gekennzeichnet, daß** $R^1$ bzw. $R^2$ für eine Gruppe $NHCO(CH_2)_2-COOH$, $NHCOCOOH$, $NHCOCH_2OCH_2COOH$, $NHCOCH_2OCH_3$, $N(CH_2COOH)COCH_2OCH_3$, $NHCOCH_3$, $N(CH_3)COCH_2OCH_3$
steht.

**8.** Iodhaltige dendrimere Polymere gemäß Anspruch 1 und 2, **dadurch gekennzeichnet, daß** V für eine Gruppe $-(CH_2)_4-$, $-CH_2-C_6H_4-CH_2-$, $-(CH_2)_2-$, $-(CH_2)_2-O-(CH_2)_2O(CH_2)_2-$, $-CH_2CHOHCH_2-$, $-(CH_2)_2-O-(CH_2)_2$steht.

**9.** Iodhaltige dendrimere Polymere gemäß Anspruch 1 und 2, **dadurch gekennzeichnet, daß** $-(CO)_q-U-COOH$ für eine Gruppe $CO(CH_2)_2-COOH$, $-COCOOH$,$-CO(CHOH)_2-COOH$, $COCH_2OCH_2COOH$, $COCH_2COOH$, $COCH(OCH_3)COOH$, $CH_2CH_2COOH$
steht.

**10.** Verfahren zur Herstellung von iodhaltigen dendrimeren Polymeren gemäß den Ansprüchen 1 und 2, **dadurch gekennzeichnet, daß** man dendrimere Polymere der allgemeinen Formel I'

$$A-(X')_b \qquad (I'),$$

worin
A und b die in den Ansprüchen 1 und 2 angegebene Bedeutung haben und
X' die für X in Anspruch 1 angegebene Bedeutung hat, wobei jedoch im Unterschied zu X für die n-te Generation die Positionen $\alpha$ nicht durch die Reste Z und gegebenenfalls $-(CO)_q-U-COOH$, sondern durch Wasserstoffatome besetzt sind, mit Verbindungen der allgemeine Formel II

$$Y'-B' \qquad (II),$$

worin

Y' für einen eine Carbonyl-, Thiocarbonyl-, aktivierte Carbonyl- oder eine CHR=CR-Gruppe - mit R in der Bedeutung eines Wasserstoffatoms oder einer Methylgruppe - enthaltenden in Y umzuwandelnden Rest steht und

B' die für B angegebene Bedeutung eines Triiodaromaten hat, wobei in B enthaltende Carboxy- und Hydroxygruppen jedoch in geschützter Form vorliegen,

umsetzt und anschließend die gegebenenfalls nicht durch die Reste Z besetzten Positionen $\alpha$ mit einem den Rest $-(CO)_q-U-COOH$ einführenden Reagenz acyliert bzw. alkyliert.

**11.** Diagnostische Mittel enthaltend mindestens ein iodhaltiges dendrimeres Polymer nach Anspruch 1 und 2 in einem physiologisch verträglichen Medium, gegebenenfalls mit den in der Galenik üblichen Zusätzen.

**12.** Verwendung von mindestens einem iodhaltigen dendrimeren Polymeren nach Anspruch 1 und 2 für die Herstellung

von Mitteln für die Röntgen-Diagnostik.

**13.** Verwendung von mindestens einem iodhaltigen dendrimeren Polymeren nach Anspruch 1 und 2 für die Herstellung von Mitteln für die Röntgen-Diagnostik von Gefäßerkrankungen.

## Claims

**1.** Iodine-containing dendrimeric polymers of the general formula I

$$A\text{-}(X)_b \qquad \text{(I)},$$

in which

A is a nitrogen-containing nucleus of base multiplicity b, where
b is the numbers 1 to 8, and
X is a radical composed of

$$\sum_{k=0}^{n-1} 2^k$$

reproduction units S and a maximum of $2^n$ imaging radicals Z, in which

n defines the number of generations and is the numbers 1 to 10,
S is a radical of the formula II

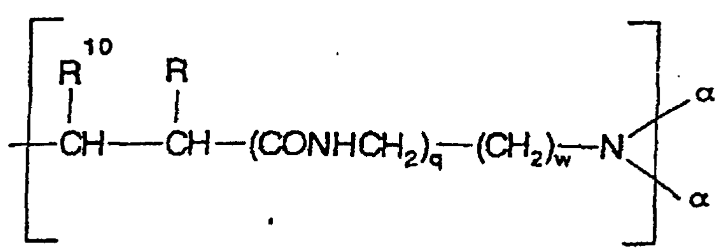

(II),

in which

R and $R^{10}$ are, independently of one another, a hydrogen atom or a methyl group,
w is the number 1 or 2,
q is the number 0 or 1, and the positions
α are $0 \leq k \leq n-1$ occupied by further reproduction units S, and for the nth generation occupied by the radicals Z, where positions α which are not occupied by Z are, where appropriate, occupied by the radicals -(CO)q-U-COOH, in which q has the abovementioned meaning, and U is a direct linkage or an alkylene chain having up to 6 C atoms, which is optionally interrupted by 1-2 oxygen atoms and/or optionally substituted by 1-4 hydroxyl groups and/or 1-2 carboxy groups,
with the proviso that a maximum of 20% of the positions α are occupied by this radical-(CO)q-U-COOH,
Z consists of an imaging radical Y-B which consists of a linker Y and of a triiodoaromatic group B, where Y or B contain at least one aliphatic carboxy, one sulpho or phosphono group and

Y is a group -CO-, -CONH-, -CSNH-,

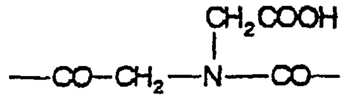, 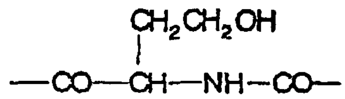,

-CHR-CHR-CONH-

or

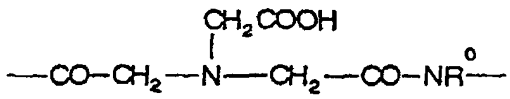

with R in the abovementioned meaning and $R^0$ meaning a hydrogen atom, a methyl or carboxymethyl group and

B is a benzene ring

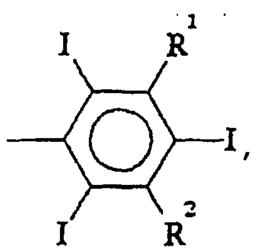

in which
$R^1$ and $R^2$ are each, independently of one another, a hydrogen atom, a $-CONR^3R^4$ or $-NR^6COR^5$ group,
where
$R^3$ and $R^4$ are, independently of one another, a hydrogen atom, a straight-chain or branched-chain or cyclic alkyl group which is optionally substituted by 1-5 hydroxyl and/or 1-3 $C_1$-$C_3$-alkoxy and/or 1-3 carboxy, sulpho or phosphono group(s) and has up to 12 C atoms,

$R^3$ and $R^4$ are, together with the nitrogen atom, a 5- or 6-membered ring which optionally contains an oxygen atom, an $SO_2$ group or an $N$-$CO$-$R^7$ radical - with $R^7$ meaning a carboxy group or an alkyl group which optionally contains 1-5 hydroxyl, 1-3 $C_1$-$C_3$-alkoxy or 1-3 carboxy, sulpho or phosphono group(s) and has up to 12 C atoms,

$R^5$ is a carboxy group, an alkyl group which is optionally interrupted by an oxygen atom and/or optionally substituted by 1-3 carboxy, sulpho or phosphono and/or 1-5 hydroxyl and/or 1-3 $C_1$-$C_3$-alkoxy group(s) and has up to 12 C atoms,

$R^6$ is a hydrogen atom, an alkyl group which is optionally substituted by 1-3 carboxy, sulpho or phosphono group(s) and/or optionally by 1-3 hydroxyl group(s) and/or 1-3 $C_1$-$C_3$-alkoxy group(s) and has up to 12 C atoms,

where the reproduction units S must be identical only for one generation,
and the salts thereof with physiologically acceptable organic and/or inorganic bases, amino acids or amino amides.

2. Iodine-containing dendrimeric polymers according to Claim 1, **characterized in that** the nucleus A is a nitrogen

atom, a radical β-$NR^8$-β, β-$NR^8R^9$ or a radical of the general formulae III, IV, V or VI,

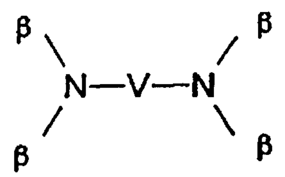

(III),

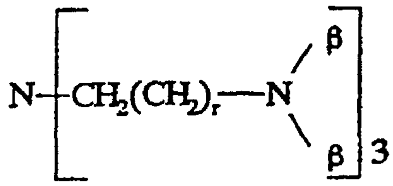

(IV),

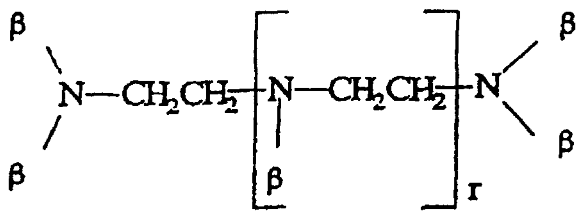

(V),

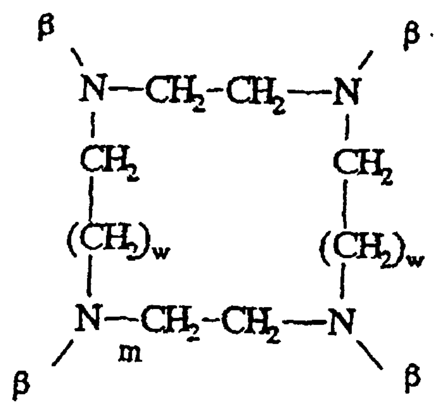

(VI),

in which

| | |
|---|---|
| $R^8$ and $R^9$ | are, independently of one another, a straight-chain or branched alkyl, aryl or aralkyl radical having up to 20 C atoms, which is optionally substituted by 1-4 hydroxyl group(s), |
| β | identifies the binding site to the radical X, where the number of β must be equal to the base multiplicity b, |
| V | is a straight-chain or branched alkylene, arylene or aralkylene radical having up to 20 C atoms, which is optionally interrupted by 1-4 oxygen atom(s) and/or substituted by 1-4 hydroxyl group(s), |
| r | is the number 1, 2 or 3, |
| w | is the number 1 or 2 and |
| m | is the number 0, 1, 2 or 3. |

3. Iodine-containing dendrimeric polymers according to Claim 1 and 2, **characterized in that** the nucleus A is a group

$\beta_2N-(CH_2)_4-N\beta_2$, $\beta_2N(CH_2)_2N\beta_2$, $\beta N(CH_2CH_2N\beta_2)_2$, $N(CH_2CH_2N\beta_2)_3$

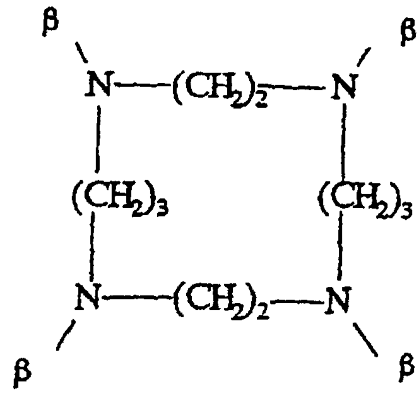

or

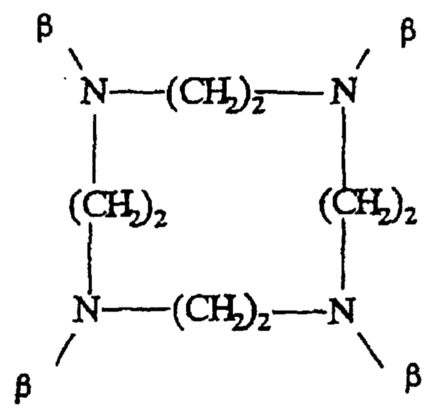

**4.** Iodine-containing dendrimeric polymers according to Claim 1 and 2, **characterized in that** S is

$-CH(CH_3)CH(CH_3)CONH-CH_2-CH_2-N$;

$-CH(CH_3)CH(CH_3)CH_2N$;

$-CH_2CH_2CH_2-N$;

$-CH_2CH(CH_3)CH_2-N$;

$-CH_2CH_2-CONH-CH_2CH_2-N$;

$-CH(CH_3)CH_2CH_2N$;

$-CH (CH_3)CH_2CONH-CH_2CH_2-N$;

$-CH_2CH(CH_3)CONH-CH_2-CH_2-N.$

**5.** Iodine-containing dendrimeric polymers according to Claim 1 and 2, **characterized in that** n is the numbers 2-6.

**6.** Iodine-containing dendrimeric polymers according to Claim 1 and 2, **characterized in that** $R^1$ or $R^2$ is a group $CONH_2$, $CONHCH_2COOH$, $CON(CH_2COOH)_2$, $CONHCH_2CH(OH)CH_2OH$, $CON(CH_3)CH_2COOH$, $CONHCH_2PO_3H_2$, $CON(CH_2PO_3H_2)_2$, $CON(CH_2COOH)CH_2PO_3H_2$, $CON(CH_3CH_2CH(OH)$ $CH_2OH$, $CONHCH_2CH_2SO_3H$, $CON(CH_2CH_2SO_3H)_2$.

**7.** Iodine-containing dendrimeric polymers according to Claim 1 and 2, **characterized in that** $R^1$ or $R^2$ is a group $NHCO(CH_2)_2-COOH$, $NHCOCOOH$, $NHCOCH_2OCH_2COOH$, $NHCOCH_2OCH_3$, $N(CH_2COOH)COCH_2OCH_3$, $NHCOCH_3$, $N(CH_3)COCH_2OCH_3$.

**8.** Iodine-containing dendrimeric polymers according to Claim 1 and 2, **characterized in that** V is a group $-(CH_2)_4-$, $-CH_2-C_6H_4-CH_2-$, $-(CH_2)_2-$, $-(CH_2)_2-O-(CH_2)_2O(CH_2)_2-$, $-CH_2CHOHCH_2-$, $-(CH_2)_2-O-(CH_2)_2-$.

**9.** Iodine-containing dendrimeric polymers according to Claim 1 and 2, **characterized in that** $-(CO)_q-U-COOH$ is a group $CO(CH_2)_2-COOH$, $-COCOOH$, $-CO(CHOH)_2-COOH$, $COCH_2OCH_2COOH$, $COCH_2COOH$, $COCH(OCH_3)$ $COOH$, $CH_2CH_2COOH$.

**10.** Process for the preparation of iodine-containing dendrimeric polymers according to Claims 1 and 2, **characterized in that** dendrimeric polymers of the general formula I'

$$A-(X')_b \quad (I')$$

in which
A and b have the meaning stated in Claims 1 and 2, and
X' has the meaning stated for X in Claim 1, but where unlike X for the nth generation the positions $\alpha$ are occupied not by the radical Z and,
where appropriate, -(CO)q-U-COOH but by hydrogen atoms, are reacted with compounds of the general formula II

$$Y'-B' \quad (II)$$

in which

Y' is a radical which contains a carbonyl, thiocarbonyl, activated carbonyl or a CHR=CR group - with R meaning a hydrogen atom or a methyl group - and which is to be converted into Y, and
B' has the meaning stated for B of a triiodoaromatic group, but where carboxy and hydroxyl groups present in B are in protected form,

and subsequently the positions $\alpha$ not occupied by the radicals Z are, where appropriate, acylated or alkylated with a reagent introducing the radical $-(CO)_q-U-COOH$.

**11.** Diagnostic compositions comprising at least one iodine-containing dendrimeric polymer according to Claim 1 and 2 in a physiologically tolerated medium, where appropriate with the additions customary in pharmaceutical technology.

**12.** Use of at least one iodine-containing dendrimeric polymer according to Claim 1 and 2 for producing compositions for X-ray diagnosis.

**13.** Use of at least one iodine-containing dendrimeric polymer according to Claim 1 and 2 for producing compositions for X-ray diagnosis of vascular disorders.

## Revendications

1. Polymères dendrimères contenant de l'iode de formule générale I

$$A\text{-}(X)_b \qquad (I),$$

où
A représente un noyau contenant de l'azote de multiplicité de base b,
b représentant les chiffres 1 à 8 et
X représentant un radical constitué de

$$\sum_{k=0}^{n-1} 2^k$$

unités de reproduction S et d'au maximum $2^n$ radicaux Z formant une image,
où
n détermine le nombre de générations et représente les chiffres 1 à 10,
S représente un radical de formule II

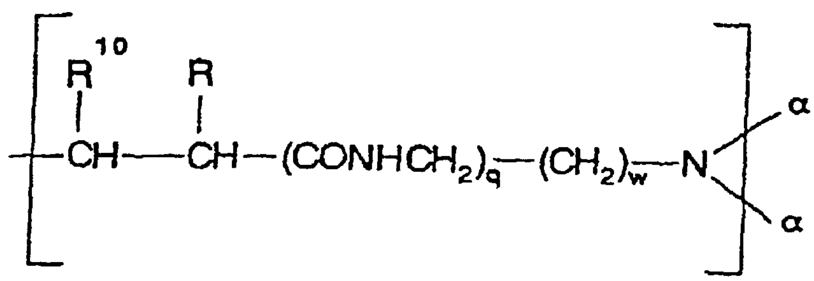

(II),

où
R et $R^{10}$ représentent, indépendamment l'un de l'autre, un atome d'hydrogène ou un groupement méthyle,
w représente les chiffres 1 ou 2,
q représente les chiffres 0 ou 1 et les positions
α sont occupées, pour 0≤k≤n-1, par d'autres unités de reproduction S et sont occupées, pour la nième génération, par les radicaux Z, les positions α qui ne sont le cas échéant pas occupées par Z étant occupées par les radicaux $-(CO)_q$-U-COOH, q ayant la signification susmentionnée et U représentant une liaison directe ou une chaîne alkylène comprenant jusqu'à 6 atomes de carbone, qui est le cas échéant interrompue par 1 à 2 atomes d'oxygène et/ou le cas échéant substituée par 1 à 4 groupements hydroxy et/ou par 1 à 2 groupements carboxy, à condition qu'au maximum 20% des positions α soient occupées par ce radical $-(CO)_q$-U-COOH,
Z est constitué d'un radical Y-B formant une image, constitué d'un élément de combinaison Y et d'un composé aromatique triiodé B, Y ou B contenant au moins un groupement aliphatique carboxy, un groupement sulfo ou phosphono et
Y représente un groupement -CO-, -CONH-, -CSNH-

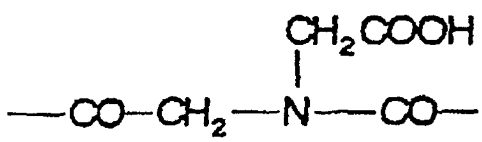 , 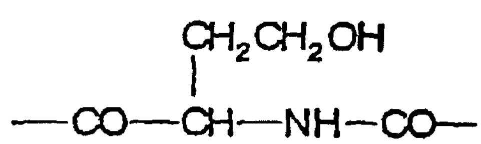 ,

-CHR-CHR-CONH-

ou

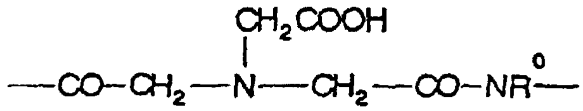

avec R ayant la signification susmentionnée et $R^0$ ayant la signification d'un atome d'hydrogène, d'un groupement méthyle ou carboxyméthyle et B représente un cycle benzène

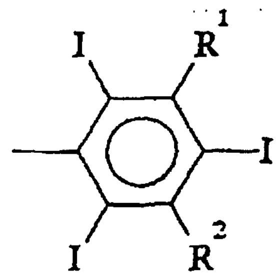

où

$R^1$ et $R^2$ signifient à chaque fois, indépendamment l'un de l'autre, un atome d'hydrogène, un groupement $-CONR^3R^4$ ou $-NR^6COR^5$,

$R^3$ et $R^4$ représentant, indépendamment l'un de l'autre, un atome d'hydrogène, un groupement alkyle linéaire ou ramifié ou cyclique, comprenant jusqu'à 12 atomes de carbone, le cas échéant substitué par 1 à 5 groupements hydroxy et/ou par 1 à 3 groupements alcoxy en $C_1$ à $C_3$ et/ou par 1 à 3 groupements carboxy, sulfo ou phosphono,

$R^3$ et $R^4$ représentant ensemble avec l'atome d'azote un cycle à 5 ou à 6 membres contenant le cas échéant un atome d'oxygène, un groupement $SO_2$ ou un radical $N-CO-R^7$ - avec $R^7$ ayant la signification d'un groupement carboxy ou d'un groupement alkyle comprenant jusqu'à 12 atomes de carbone, contenant le cas échéant 1 à 5 groupements hydroxy, 1 à 3 groupements alcoxy en $C_1$ à $C_3$ ou 1 à 3 groupements carboxy, sulfo ou phosphono,

$R^5$ représentant un groupement carboxy, un groupement alkyle comprenant jusqu'à 12 atomes de carbone, le cas échéant interrompu par un atome d'oxygène et/ou le cas échéant substitué par 1 à 3 groupements carboxy, sulfo ou phosphono et/ou par 1 à 5 groupements hydroxy et/ou par 1 à 3 groupements alcoxy en $C_1$ à $C_3$,

$R^6$ représentant un atome d'hydrogène, un groupement alkyle comprenant jusqu'à 12 atomes de carbone, le cas échéant substitué par 1 à 3 groupements carboxy, sulfo ou phosphono et/ou le cas échéant par 1 à 3 groupements hydroxy et/ou par 1 à 3 groupements alcoxy en $C_1$ à $C_3$, les unités de reproduction S ne devant être identiques que pour une génération,

ainsi que leurs sels avec des bases, des acides aminés ou des amides d'acides aminés organiques et/ou inorganiques physiologiquement sans risque.

2. Polymères dendrimères contenant de l'iode selon la revendication 1, **caractérisés en ce que** le noyau A représente un atome d'azote, un radical $\beta-NR^8-\beta$, $\beta-NR^8R^9$ ou un radical des formules générales III, IV, V ou VI,

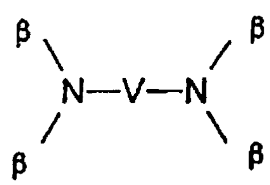

(III),

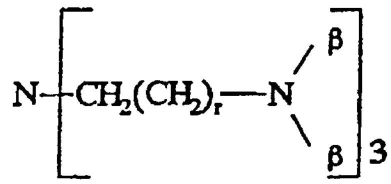

(IV),

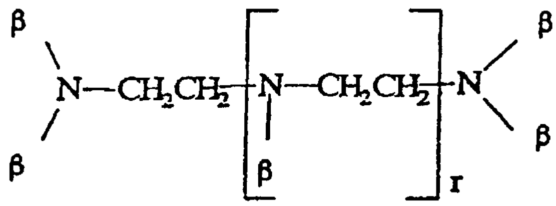

(V),

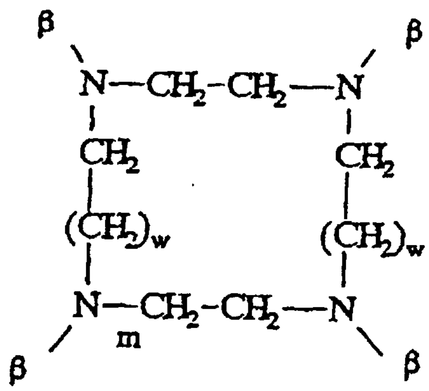

(VI),

où
$R^8$ et $R^9$ représentent, indépendamment l'un de l'autre, un radical alkyle, aryle ou aralkyle, linéaire ou ramifié, comprenant jusqu'à 20 atomes de carbone, le cas échéant substitué par 1 à 4 groupements hydroxy,
β marque l'endroit de liaison au radical X, le nombre de β étant à égaler avec la multiplicité de base b,
V représente un radical alkylène, arylène ou aralkylène linéaire ou ramifié comprenant jusqu'à 20 atomes de carbone, qui est le cas échéant interrompu par 1 à 4 atomes d'oxygène et/ou substitué par 1 à 4 groupements hydroxy,
r représente les chiffres 1, 2 ou 3,
w représente les chiffres 1 ou 2 et
m représente les chiffres 0, 1, 2 ou 3.

3. Polymères dendrimères contenant de l'iode selon les revendications 1 et 2, **caractérisés en ce que** le noyau A représente un groupement $\beta_2N(CH_2)_4N\beta_2$, $\beta_2N(CH_2)_2N\beta_2$, $\beta N(CH_2CH_2N\beta_2)_2$, $N(CH_2CH_2N\beta_2)_3$,

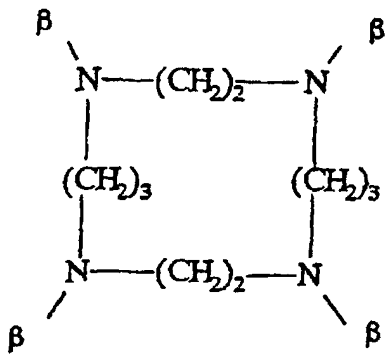

ou

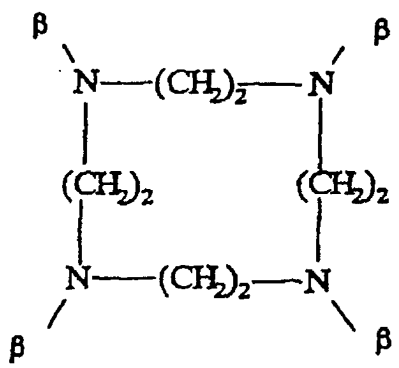

**4.** Polymères dendrimères contenant de l'iode selon les revendications 1 et 2, **caractérisés en ce que** S représente

$-CH(CH_3)CH\ (CH_3)CONH-CH_2-CH_2-N$ ;

$-CH(CH_3)CH(CH_3)CH_2N$ ;

$-CH_2CH_2CH_2-N$ ;

$-CH_2CH(CH_3)CH_2-N$ ;

$-CH_2CH_2-CONH-CH_2CH_2-N$ ;

$-CH(CH_3)CH_2CH_2N$ ;

$-CH(CH_3)CH_2CONH-CH_2CH_2-N$ ;

$-CH_2CH(CH_3)CONH-CH_2-CH_2-N$.

**5.** Polymères dendrimères contenant de l'iode selon les revendications 1 et 2, **caractérisés en ce que** n représente les chiffres 2 à 6.

**6.** Polymères dendrimères contenant de l'iode selon les revendications 1 et 2, **caractérisés en ce que** $R^1$ ou, selon le cas, $R^2$ représente un groupement $CONH_2$, $CONHCH_2COOH$, $CON(CH_2COOH)_2$, $CONHCH_2CH(OH)CH_2OH$, $CON(CH_3)CH_2COOH$, $CONHCH_2PO_3H_2$, $CON(CH_2PO_3H_2)_2$, $CON(CH_2COOH)CH_2PO_3H_2$, $CON(CH_3)CH_2CH(OH)CH_2OH$, $CONHCH_2CH_2SO_3H$, $CON(CH_2CH_2SO_3H)_2$.

**7.** Polymères dendrimères contenant de l'iode selon les revendications 1 et 2, **caractérisés en ce que** $R^1$ ou, selon le cas, $R^2$ représente un groupement $NHCO(CH_2)_2$-COOH, NHCOCOOH, $NHCOCH_2OCH_2COOH$, $NHCOCH_2OCH_3$, $N(CH_2COOH)COCH_2OCH_3$, $NHCOCH_3$, $N(CH_3)COCH_2OCH_3$.

**8.** Polymères dendrimères contenant de l'iode selon les revendications 1 et 2, **caractérisés en ce que** V représente un groupement $-(CH_2)_4-$, $-CH_2-C_6H_4-CH_2-$, $-(CH_2)_2-$, $-(CH_2)_2-O-(CH_2)_2O(CH_2)_2-$, $-CH_2CHOHCH_2-$, $-(CH_2)_2-O-(CH_2)_2-$.

**9.** Polymères dendrimères contenant de l'iode selon les revendications 1 et 2, **caractérisés en ce que** $-(CO)_q$-U-COOH représente un groupement $CO(CH_2)_2$-COOH, -COCOOH, $-CO(CHOH)_2$-COOH, $COCH_2OCH_2COOH$, $COCH_2COOH$, $COCH(OCH_3)COOH$, $CH_2CH_2COOH$.

**10.** Procédé pour la préparation de polymères dendrimères contenant de l'iode selon les revendications 1 et 2, **caractérisé en ce qu'**on transforme des polymères dendrimères de formule générale I'

$$A\text{-}(X')_b \qquad (I'),$$

où
A et b ont la signification indiquée dans les revendications 1 et 2 et
X' a la signification indiquée pour X dans la revendication 1, toutefois, à la différence de X, les positions $\alpha$ pour la nième génération n'étant pas occupées par les radicaux Z et le cas échéant $-(CO)_q$-U-COOH, mais par des atomes d'hydrogène,
avec des composés de formule générale II

$$Y'\text{-}B' \qquad (II),$$

où
Y' représente un radical à transformer en Y contenant un groupement carbonyle, thiocarbonyle, carbonyle activé ou CHR=CR - avec R ayant la signification d'un atome d'hydrogène ou d'un groupement méthyle - et
B' a la signification indiquée pour B d'un composé aromatique triiodé, les groupements carboxy et hydroxy contenus dans B se trouvant toutefois sous forme protégée
et qu'on acyle ou, selon le cas, alkyle ensuite les positions $\alpha$ qui ne sont le cas échéant pas occupées par les radicaux Z avec un réactif introduisant le radical - $(CO)_q$-U-COOH.

**11.** Agent diagnostique contenant au moins un polymère dendrimère contenant de l'iode selon les revendications 1 et 2 dans un milieu physiologiquement compatible, le cas échéant avec des additifs usuels en galénique.

**12.** Utilisation d'au moins un polymère dendrimère contenant de l'iode selon les revendications 1 et 2 pour la préparation d'agents pour le diagnostic par rayons X.

**13.** Utilisation d'au moins un polymère dendrimère contenant de l'iode selon les revendications 1 et 2 pour la préparation d'agents pour le diagnostic par rayons X de maladies vasculaires.